# EUROPEAN PATENT SPECIFICATION

(11) **EP 1 558 934 B1**
(45) Date of publication and mention of the grant of the patent: **17.07.2013**
(21) Application number: 03809704.4
(22) Date of filing: 31.10.2003
(51) Int. Cl.: G01N 33/58, G01N 33/543

(54) **A METHOD FOR ASSESSMENT OF PARTICLES**
VERFAHREN ZUM NACHWEIS VON PARTIKELN
PROCEDE D'EVALUATION DE PARTICULES

(30) Priority: 31.10.2002 DK 200201653
(43) Date of publication of application: 03.08.2005
(73) Proprietor: ChemoMetec A/S, 3450 Allerød (DK)
(72) Inventor: LARSEN, Rasmus, Dines, DK-2000 Frederiksberg (DK); HANSEN, Frans, Ejner, Ravn, DK-1905 Frederiksberg (DK)
(74) Representative: Høiberg A/S
(86) International application number: PCT/DK2003/000743
(87) International publication number: WO 2004/040314

(56) References cited:
- EP-A2- 1 180 675
- WO-A-00/28297
- WO-A-02/08754
- WO-A-96/22531
- WO-A-98/50577
- WO-A-99/49315
- WO-A2-00/11024
- DIETZ L.J. ET AL: "Volumetric capillary cytometry: a new method for absolute cell enumeration.", CYTOMETRY, vol. 23, no. 3, 1 March 1996 (1996-03-01), pages 177-186, XP001096327,
- GALBRAITH W. ET AL: "IMAGING CYTOMETRY BY MULTIPARAMETER FLUORESCENCE", CYTOMETRY, vol. 12, 1 January 1991 (1991-01-01), pages 579-596, XP000942723,
- WITTRUP K. ET AL: "Fluorescence array detector for large-field quantitative fluorescence cytometry.", CYTOMETRY, vol. 16, no. 3, 1 July 1994 (1994-07-01), pages 206-213,

## Description

### Field of invention

The present invention relates to methods for the assessment of particles, based on imaging of particles with less than 10⁶ analyte detectable positions contained within the particles or on the surface of the particles.

### Background of invention

Detection of a substance or a particle using staining of the substance or particle to aid detection is widely used. However, many substances and particles are so small that although stained it is difficult to detect them without using very high magnification increasing the requirements to the equipment used and reducing the volume of sample examined at one time.

Most methods for detection of particles based on imaging a volume of a sample containing the particles onto e.g. a CCD are based on addition of some sort of stain to the particle. In the case of cells a particularly popular type of stain is a stain capable of binding to DNA present in the cells. The reason for this is the abundance of DNA in cells. In human cells, there are approximately 3x10⁹ DNA bases in every cell. Thus the theoretical number of positions to which a molecular stain can be bound is extremely high. Under such conditions the amount of electromagnetic radiation is so high that the exposure times can be low (in flow cytometry, the acquisition rate is typically 5,000 to 10,000 per second) and an acceptable signal/noise ratio can be achieved.

A classical amplification technique is that of enzyme-linked assay. A ligand reacting specifically with the analyte is bound to an enzyme, and after excess ligand-enzyme is removed, the analyte-ligand-enzyme complex is detected by reaction with a chromogenic substrate, a colourless material which is acted upon by the enzyme to form a coloured product. Because of its large amplification factor, enzyme-linked assays offer high sensitivity, and are particularly useful for detection of small amounts of antigens.

WO 98/50777 and WO 00/28297 (CHEMOMETEC) disclose methods and systems for assessment of properties of particles in a liquid sample. According to these disclosures the assessment is performed by staining the particles with stains which are known to stain DNA monomers. Accordingly these references disclose methods for assessment based on staining abundant molecules in the cells. Under such conditions the signal accumulated from each cell is relatively high and the signal to noise ratio is correspondingly low so that distinction between signal from particles and background is facilitated.

The patent application WO 00/11024 discloses an architecture for microvolume laser-scanning cytometers, wherein several laser spots scan the sample. A binned CCD detector used in non-imaging mode then simultaneously collects data from the regions exposed by the laser spots. Alternatively, Photomultiplier Tubes are used for collecting data.

The article "Volumetric Capillary Cytometry: A new method for absolute cell enumeration" by Dietz et al. published in Cytometry, vol. 23, pp 177-186 in 1996 relates to a cytometry system for imaging, identifying and counting fluorescently labelled cells and other particles using photomultiplier tubes to detect the signal from the fluorescent markers. The cytometry system comprises a "focusing lens" placed immediately above the sample for linear enlargement of the representation of electromagnetic signals from the sample. Furthermore, the article describes the detection of CD3+ and CD4+ lymphocytes.

The article "Imaging cytometry by multiparameter fluorescence" by Galbraith W. et al published in cytometry, vol. 12, pp 579-596 in 1991 relates to a method for assessing CD4, CD14 and CD8 antigens on lymphocytes using fluorescently labelled antibodies, arranging the sample on a microscope slide and detecting the signal at 20x magnification.

The article "Fluorescence array detector for large-field quantitative fluorescence cytometry" by wittrup K et al. published in Cytometry, vol 16, pp 206-213 in 1994 relates to analysis of weakly fluorescent particles at no magnification in dried form on a microscope slide.

It is an object of the present invention to provide an alternative method for the assessment of properties related to particles based on staining of analytes which are several orders of magnitude less abundant than DNA monomers.

### Summary of invention

In a first aspect the invention relates to a method for assessing at least one quality parameter or at least one quantity parameter of a particle in a liquid material according to claim 1, said liquid material comprising particles having bound thereto or comprised therein at least one species of analytes in an amount of less than 1x10⁶ analyte detectable positions,
comprising:
mixing the liquid material with at least one reagent material, said reagent material at least comprising a first targeting species capable of selectively and directly binding to an analyte position of said species of analytes, and a labelling agent, wherein the first targeting species and the labelling agent are directly or indirectly coupled to each other,
arranging a volume of a liquid material comprising at least part of the mixture of the liquid material and the reagent material in a sample compartment having a wall part defining an exposing area, the wall part allowing electromagnetic signals from the sample in the compartment to pass through the wall to the exterior,
exposing, onto an array of active detection elements, a representation of electromagnetic signals having passed through the wall part from the sample in the sample compartment, wherein the representation is subject to a linear enlargement, so that the ratio of the image of a linear dimension on the array of detection elements to the original linear dimension in the exposing domain is smaller than 20:1,
detecting the representation as intensities by individual active detection elements,
processing the intensities in order to identify representations of electromagnetic signals from the particles as distinct from representations of electromagnetic signals from background, and
obtaining the at least one quantity parameter or at least one quality parameter of the particles from the result of the processing.

The step of exposing and detecting electromagnetic signals corresponds to recording an image of the sample in the sample compartment. What is detected by the detection elements is a representation of electromagnetic signals.

Surprisingly it has turned out that it is possible to process the intensities and identify signals from particles as distinct from signals from background even when so few analytes in the particles are stained and contribute to the electromagnetic radiation from the particles. In the appended examples it has been demonstrated that it is possible to assess the percentage of CD3+ (CD3 positive), CD4+, CD8+ and CD45+ leukocytes using PE (R-phycoerythrin) conjugated antibodies in accordance with the method of the present invention, under conditions which are readily applicable to routine analysis using less complex apparatuses than generally applied in similar tasks.

### Description of Drawings

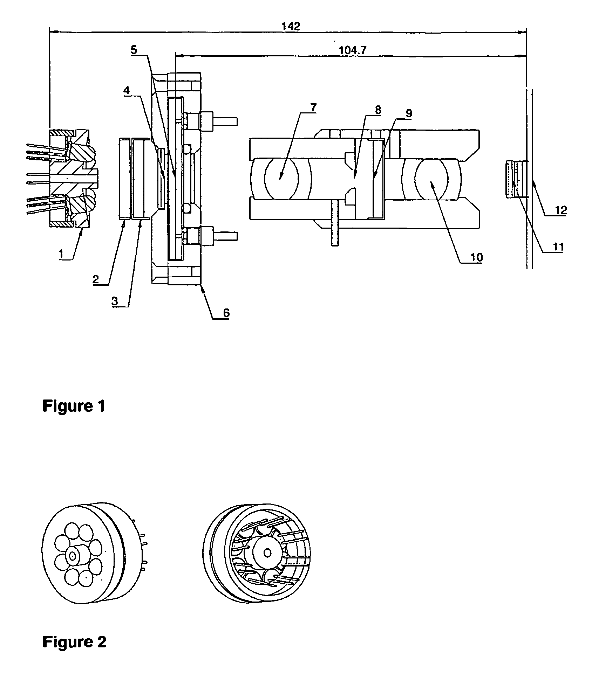

**Figure 1****.** Schematic diagram of the experimental "double triplet system". A microscopic ChemoMetec module was modified by changing and moving the lenses and optical components in order to obtain larger numerical aperture (NA= approx. 0.10) having a low transversal magnification (M_{T}=0.98) and by changing the filters. A flow cuvette with 40 µm of spacing was used and the cuvette flow path was 8 mm wide. Distance unit is mm. Components are listed in Table 1 below.
Figure 2. Perspective drawing of the LED fixture with 8 LEDs showing front (left) and back that is mounted towards the LED PCB (right). Diameter is approximately 30 mm.

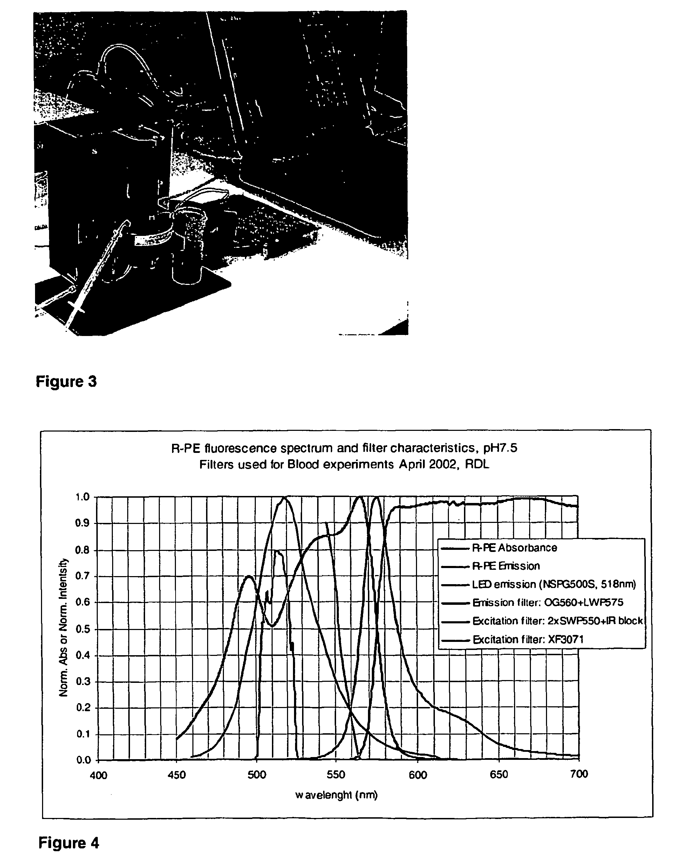

**Figure 3****.** Picture of the experimental set-up. A microscopic module (left) was modified by changing the lenses in order to obtain larger numerical aperture (NA= approx. 0.10) having a low transversal magnification (M_{T}=0.98) and by changing the filters. The counting of the fluorescent objects was conducted both during analysis by the module (left) and after analysis by PC software (based on LabVIEW, National Instruments).
**Figure 4****.** Optical characteristics for the filters and the spectrum for R-PE (R-phycoerythrin) and the LEDs. The filters have been optimised for detection of light emitted from R-PE.

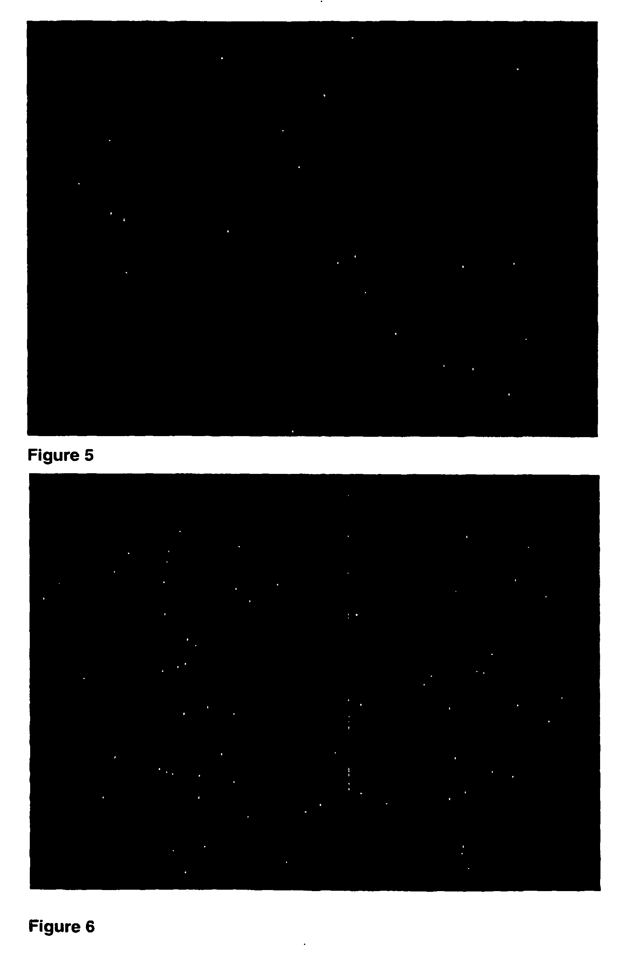

Figure 5. Image (screen dump, example) from the detection and quantification of human peripheral blood cells stained with anti-CD8/PE conjugated antibodies (as described in the "no wash" procedure, except dilution step 5 omitted ). The image corresponds to a volume of approx. 0.75 *µ*L which corresponds to approx. 0.02 *µ*L of undiluted whole blood when the "no wash" protocol described is used.
**Figure 6****.** Image from the detection and quantification of QuantiBritePE beads diluted in IgG1/PE stained control blood sample. IgG1/PE stained control blood sample was pre-diluted in PBS buffer with 0.5% Pluronic PE3100 as described in the "no wash" procedure prior to dissolving the beads. The image corresponds to a volume of approx. 0.75 *µ*L which corresponds to approx. 0.02 *µ*L of undiluted whole blood when the "no wash" protocol described is used.

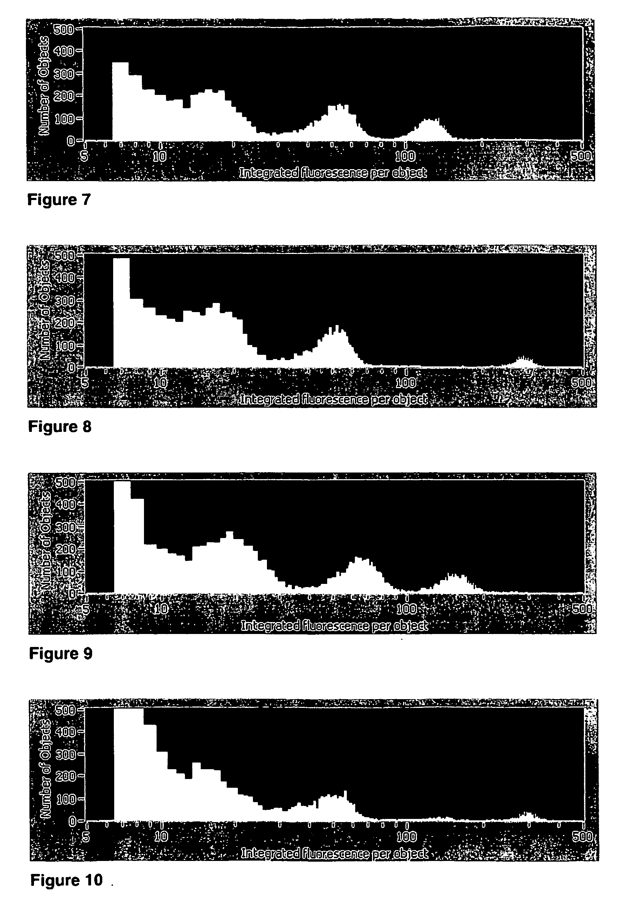

**Figure 7****.** QuantiBritePE beads (lot. 32417) in PBS with 0.5% Pluronic PE3100 are plotted into a histogram according to their individual integrated fluorescence intensities. Total approx. 10,500 events have been imaged, whereof approx. 9,500 events are beads (three populations detected). Objects having sizes of 1 pixel and IFI (Integrated Fluorescence Intensity) <10 are objects that are very close to the detection limit (background). Threshold =7. Populations geometric mean IFI are approx. 120, 53, 18 (populations from right to left) and the bead populations contain approximately 78,000, 37,000, and 13,400 PE molecules per bead (mean) (populations from right to left).
**Figure 8****.** QuantiBritePE beads (lot. 20977) in PBS with 0.5% Pluronic PE3100 are plotted into a histogram according to their individual integrated fluorescence intensities (IFI). Total 10,192 events have been imaged, whereof approx. 9,500 events are beads. Objects having sizes of 1 pixel and IFI <10 are objects that are very close to the detection limit (background). Threshold =7. Populations geometric mean IFI are approx. 290, 51, 19 (populations from right to left) and the bead populations contain approximately 182,000, 36,600, and 14,000 PE molecules per bead (mean) (populations from right to left).
**Figure 9****.** QuantiBritePE beads (lot. 32417) in IgG1/PE control blood sample (diluted in PBS with 0.5% Pluronic PE3100 as described in the "no wash" procedure) are plotted into a histogram according to their individual integrated fluorescence intensities (IFI). Total approx. 10,002 events have been imaged, whereof approx. 9,200 events are beads. Objects having sizes of 1 pixel and IFI <10 are objects that are very close to the detection limit (background). Threshold =7. Populations geometric mean IFI are approx. 150, 62, 20 (populations from right to left), and the bead populations contain approximately 78,000, 37,000, and 13,400 PE molecules per bead (mean) (populations from right to left).
**Figure 10****.** QuantiBritePE beads (lot. 20977) in IgG1/PE control blood sample (diluted in PBS with 0.5% Pluronic PE3100 as described in the "no wash" procedure) are plotted into a histogram according to their individual integrated fluorescence intensities (IFI). Total approx. 10,500 events have been imaged, whereof approx. 9,500 events are beads. Objects having sizes of 1 pixel and IFI <10 are objects that are very close to the detection limit (background). Threshold =7. Populations geometric mean IFI are approx. 305, 53, 18 (populations from right to left) and the bead populations contain approximately 182,000, 36,600 and 14,000 PE molecules per bead (mean) (populations from right to left).

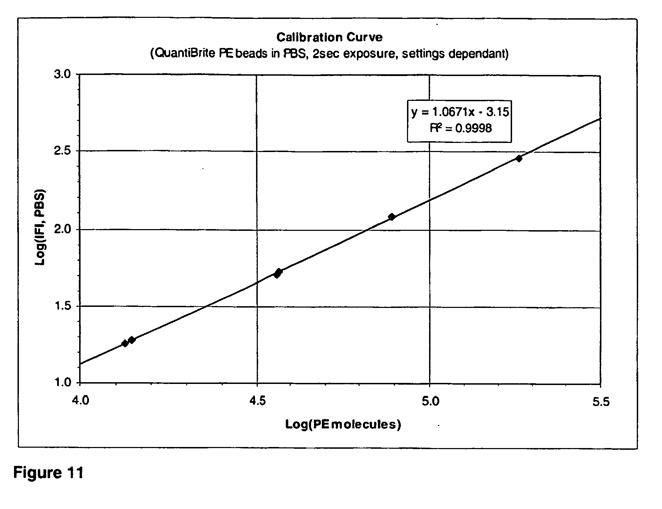

**Figure 11****.** Calibration curve using data from Table 2. QuantiBritePE beads in PBS buffer with 0.5% PE3100.

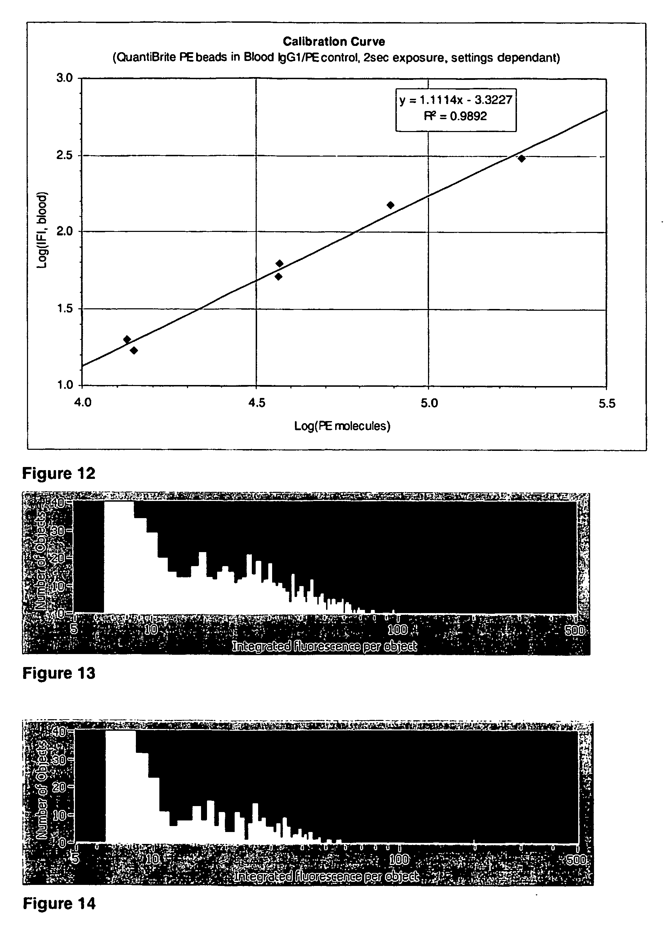

**Figure 12****.** Calibration curve using data from Table 2. QuantiBritePE beads diluted in pre-diluted IgG1/PE stained blood. The IgG1/PE immunostained blood was prediluted in PBS with 0.5% PE3100 as described in the "no wash" procedure.
**Figure 13****.** CD3+ cells are plotted into a histogram according to their integrated fluorescence intensities (IFI). Approx. 824 objects were detected whereof approx. 480 appeared as CD3+ cells. Sum of 40 images, corresponding to approx. 0.625 µL of whole blood being analysed. Settings have not been optimised for the individual staining procedure but are the same as in Figure 2 and 3. The anti-CD3/PE immunostained blood was prediluted in PBS with 0.5% Pluronic PE3100 as described in the "no wash" procedure.
**Figure 14****.** CD4+ cells are plotted into a histogram according to their integrated fluorescence intensities (IFI). Approx. 387 objects were detected whereof approx. 202 appear as CD4+ cells. Sum of 20 images, corresponding to approx. 0.313 µLof blood being analysed. Settings have not been optimised for the individual staining procedure but are the same as in Figure 2 and 3. The anti-CD4/PE immunostained blood was prediluted in PBS with 0.5% Pluronic PE3100 as described in the "no wash" procedure.

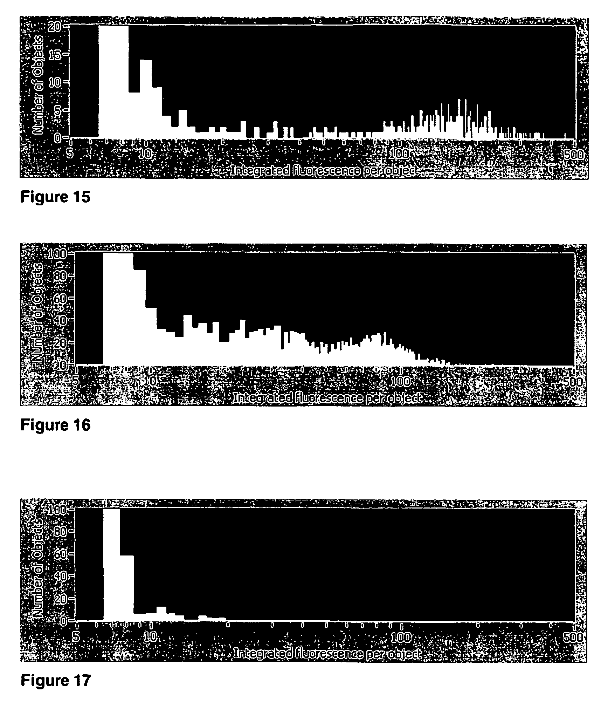

**Figure 15****.** CD8+ cells are plotted into a histogram according to their integrated fluorescence intensities (IFI). Approx. 523 objects were detected whereof approx. 410 appear as CD8+ cells. Sum of 20 images, corresponding to approx. 1.25 µL of blood being analysed. Settings have not been optimised for the individual staining procedure but are the same as in Figure 2 and 3. The anti-CD8/PE immunostained blood was undiluted (step 5. omitted) in the "no wash" procedure.
**Figure 16****.** CD45+ cells are plotted into a histogram according to their individual integrated fluorescence intensities. Approximately 4,100 objects were detected whereof approx. 2,200 appeared as CD45+ cells. Sum of 40 images, corresponding to approx. 0.625 µL of blood being analysed. Settings have not been optimised for the individual staining procedure but are the same as in Figure 2 and 3. The anti-CD45/PE immunostained blood was prediluted in PBS with 0.5% Pluronic PE3100 as described in the "no wash" procedure.
**Figure 17****.** Mouse isotype control IgG1/PE. Objects are plotted into a histogram according to their individual integrated fluorescence intensities. Total 280 objects were detected whereof only 10-20 objects appear as cells. Sum of 20 images, corresponding to approx. 0.313 µL of blood being analysed. Settings have not been optimised for the individual staining procedure but are the same as in Figure 2 and 3. Most of the objects having IFI>10 are hot-pixels that may be corrected by calibration of the CCD. The IgG1/PE immunostained blood was prediluted in PBS with 0.5% Pluronic PE3100 as described in the "no wash" procedure.

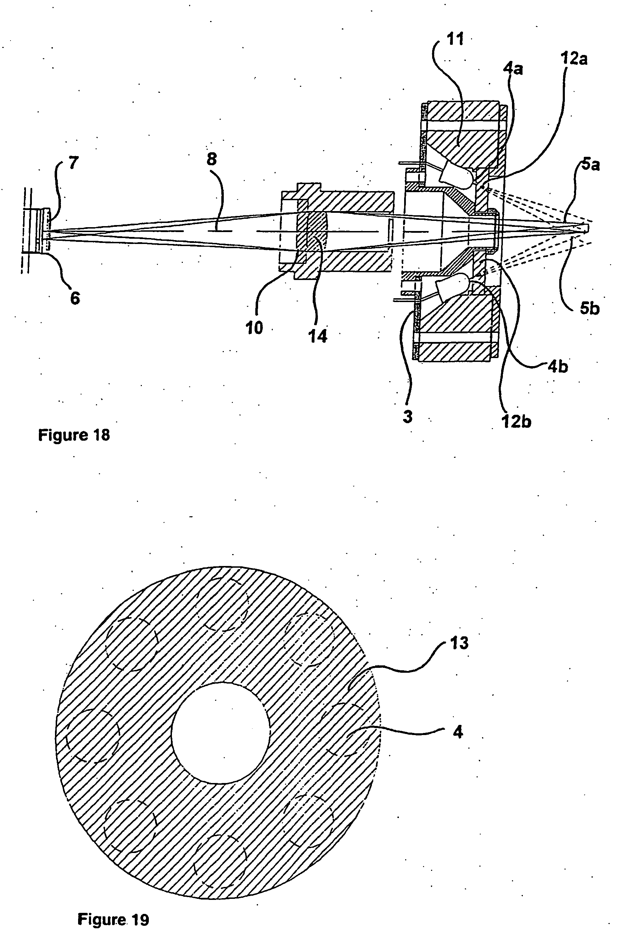

**Figure 18** shows a one sided excitation system.
**Figure 19** shows a cross-section of the excitation light filter in a plane parallel to the sample plane.

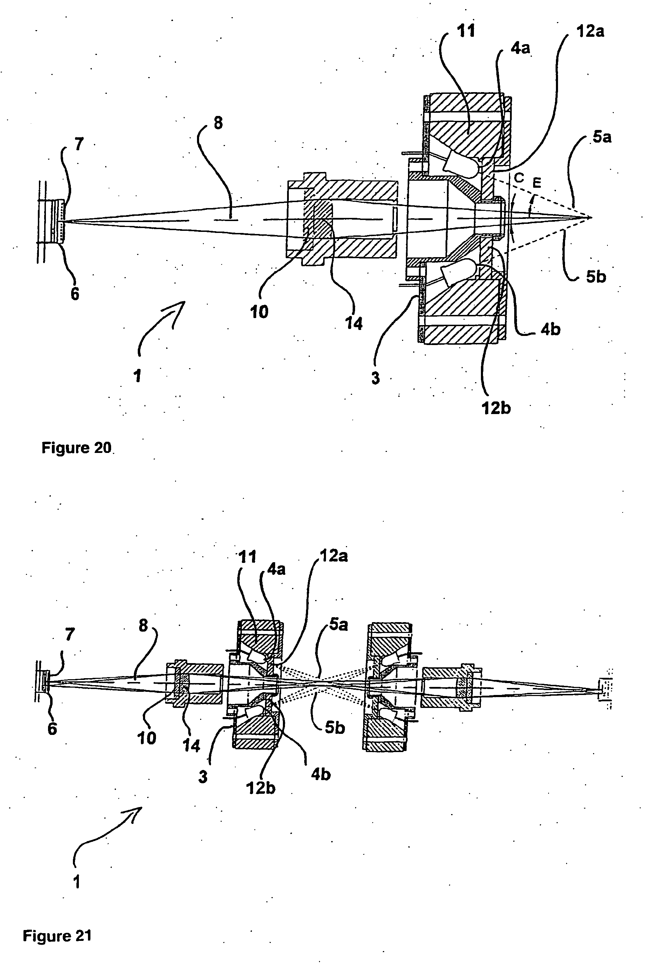

**Figure 20** shows the collection angle C and the angle E between the excitation main light path and the detection -sample axis.
**Figure 21** shows a double-sided excitation/detection system.

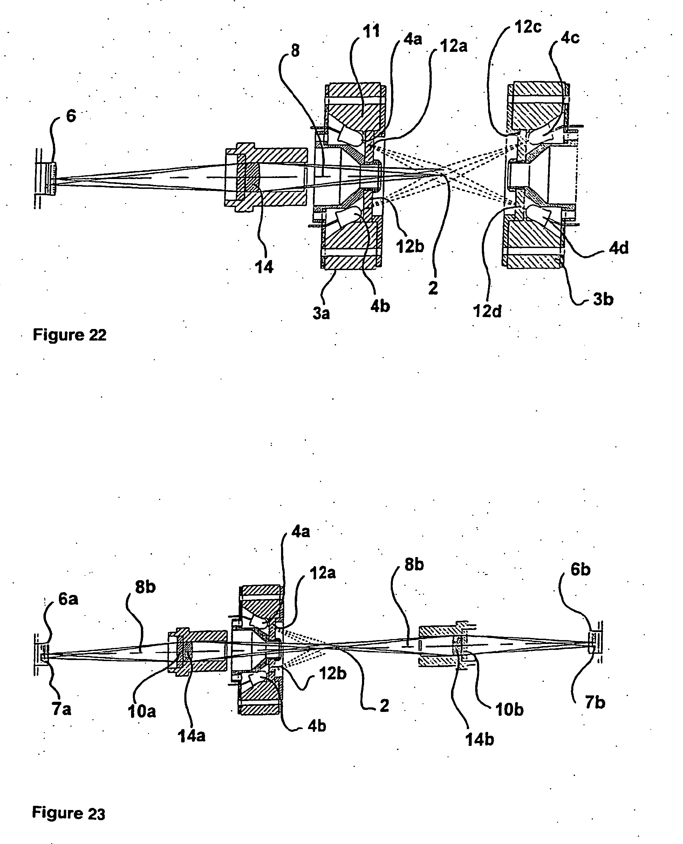

**Figure 22** shows a double-sided excitation system.
**Figure 23** shows a double-sided detection system.

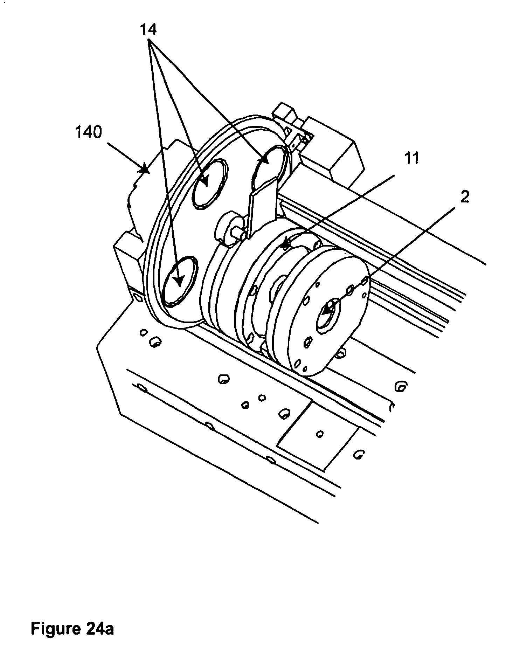

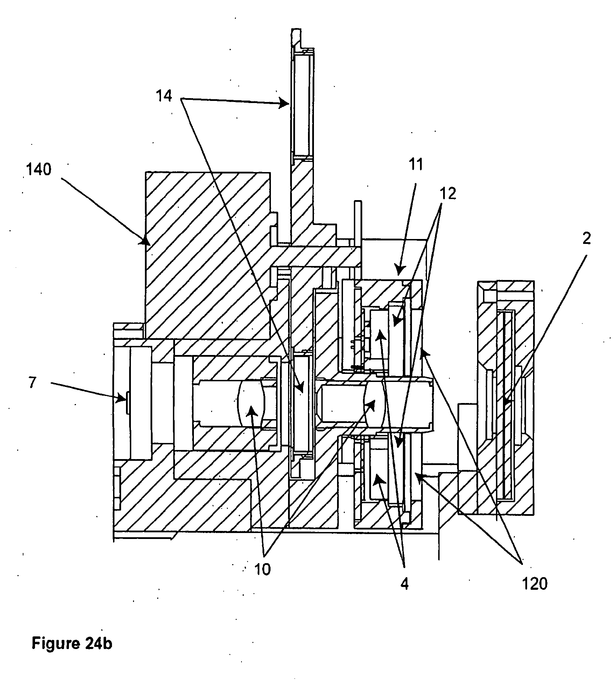

**Figure 24** **(a** and **b)** shows a double-sided detection system with interchangeable emission filters.

### Detailed description of the invention

### Definitions

Quality parameter: a parameter or a property of a single particle or a group of particles, often used for the identification of particles, generally associated with the identification of a substantially specific particle or a specific property of a particle. Also a quality parameter can be a measure of an amount of a certain property of a specific particle or species of particles, generally with the purpose of identifying, or distinguishing between different types of particles or to distinguish between two or more states a particle represent, such as growth or metabolic states.

Quantity parameter: a parameter or a property of a single particle or a group of particles often used for the enumeration of particles or relative enumeration of two or more species or groups of particles.

Statistical Quality: a measure of a random property or behaviour, generally relating to precision and/or accuracy, such as repeatability and reproducibility. The level of quality, or relative level of quality are generally expressed in terms of variation relative to expected value (e.g. standard deviation of a normal distribution) or as reliability (e.g. reliability in discrimination or classification).

Species of analyte: a compound on the surface of a particle or within a particle, to which at least one targeting species can be bound.

Analyte detectable position: a position within a species of analyte to which one molecule of a targeting species can bind (either covalently, through affinity binding (antibodies, hybridisation), or by hydrogen bonding)

Targeting species: a compound capable of binding to an analyte detectable position and directly or indirectly binding a labelling agent thereto through binding of a labelling agent to the targeting species. The coupling between the targeting species and the labelling agent may be direct or indirect (see below).

Labelling agent: a compound capable of emitting, absorbing, attenuating or scattering electromagnetic radiation to result in the generation of a detectable electromagnetic signal.

Directly coupled: the coupling refers to the coupling between the targeting species and the labelling agent. Directly coupled means that the targeting species and the labelling agent are bound by a covalent bond and thus form one compound.

Indirectly coupled: the coupling refers to the coupling between the targeting species and the labelling agent. Indirectly coupled means that the targeting species and the labelling agent are coupled via a non-covalent bond, e.g. an affinity bond (antibody-antigen, antibody-hapten, nucleotide hybridisation). In this case the targeting species and the labelling agent are two distinct compounds.

### Particles

The particles are preferably biological particles. Biological particles are in particular selected from the group consisting of cells, cell walls, bacteria, plasmodia, virus, prions, macromolecules, proteins, polypeptides, peptides, genes, DNA, RNA, or fragments or clusters thereof. Preferably, the biological particles are cells.

The cells are preferably selected from mammalian cells, insect cells, reptile cells, fish cells, yeast cells, and fungi cells, more preferably from blood cells, sperm cells, and bone marrow cells.

The particles may be coupled to a solid support, such as beads, said beads being capable of being suspended in the sample domain. The beads may be polymer beads. Often the polymer beads can have physical and/or chemical properties which can assist in the handling of the particles such as paramagnetic beads.

One embodiment of the present invention which involves the assessment of virus or other small particle is based on binding of the particle to a bead. This allows the particle to be treated in a more simple manner during pre-treatment such as with centrifugation, filtration or magnetic separation.

The beads may be labelled themselves to improve accuracy in the identification of one or more particles. One embodiment of the present invention uses two or more types of beads which can have affinity to bind two or more different particles.

Also, the particle may be a DNA or RNA containing particle whereby the DNA/RNA or a fraction thereof may be stained with a DNA staining compound. This is often preferred when a further confirmation or assessment of particle property or particle specificity is needed such as assessment of viability.

### Analytes and analyte detectable positions

According to the present invention a species of analyte is defined as a compound on the surface of a particle or within a particle, to which at least one targeting species can be bound. Examples of species of analytes include proteins, nucleotides, nucleic acid sequences, lipids, membrane constituents.

An analyte detectable position is a position within a species of analyte to which one molecule of a targeting species can bind (either covalently, through affinity binding (antibodies, hybridisation), or hydrogen bonding). One species of analyte can thus contain more than one analyte detectable position. When the species of analyte is a protein which can be targeted by an antibody, each protein molecule may contain more than one epitope to which an antibody can bind.

More specifically, the analyte may be selected from proteins, polypeptides, peptides, lipids, carbohydrates, lipoproteins, carbohydrate-conjugated proteins, membrane constituents, receptors, genes, DNA, RNA, or fragments or clusters thereof.

The analyte may be bound to a cell membrane or a cell wall or to a cell nucleus membrane. This is more particularly so when the analyte is a receptor, e.g. a cell surface receptor.

The analyte may either be located on the cell surface or be contained within the cell. The analyte may also be located on the surface of an organelle or be contained within an organelle.

The total number of analyte detectable positions in a particle is determined by multiplying the number of the particular species of analyte by the number of analyte detectable positions in each species of analyte.

The particles may have bound thereto or comprised therein at least one species of analytes in an amount of less than 1x10⁶ analyte detectable positions, for example less than 5x10⁵ analyte detectable positions, such as less than 1x10⁵ analyte detectable positions, such as less than 5x10⁴ analyte detectable positions, such as less than 1x10⁴ analyte detectable positions, such as less than 5x10³ analyte detectable positions, such as less than 1x10³ analyte detectable positions, such as less than 5x10² analyte detectable positions, such as less than 1x10² analyte detectable positions, such as less than 10 analyte detectable positions, such as 1 or 2 analyte detectable position. The lower the number of analyte detectable positions the less signal can be produced by each particle so that it becomes increasingly difficult to identify the particles as such in the image of the sample domain.

The present invention is particularly adapted for assessing particles which have between 250 and 250,000 analyte detectable positions (average for population), more preferably between 500 and 50,000 analyte detectable positions, even more preferably between 1,000 and 10,000 analyte detectable positions bound thereto or comprised therein.

The following table gives the number of different membrane antigens per cell for various human cells. The membrane antigens are examples of species of analyte.

| Membrane Antigen | Cell type | Antigen Density per Cell | Reference |
|---|---|---|---|
| | | Mean ± SD | |
| | | (x 10³) | |
| CD3 | T cells | 57 ± 7 | 1 |
| CD4 | T_{H} cells | 47 ± 14 | 1 |
| | Monocytes | 17 ±5 | |
| CD8 | T_{C} cells | 145 ± 29 | 1 |
| | NK cells | 39 ± 10 | |
| CD19 | B cells | 27 ± 3 | 1 |
| CD23 | B **cells** | 4.4 | 2 |
| CD45 | Lymphocytes | 217 ± 64 | 1 |
| | Monocytes | 103 ± 44 | |
| | Granulocytes | 36 ± 16 | |

| | | | |
|---|---|---|---|
| References: 1. Bikoue A., et al., Quantitative Analysis of Leukocyte Memebrane Antigen Expression: Normal Adult Values, Cytometry, 26, p.137-47, (1996) 2. Lenkei R., Determination of the antibody binding capacity of lymphocyte membrane antigen binding capacity by flow cytometry in 58 blood donors, Journal of Immunogical Methods, 183, p.267-77, (1995) | | | |

Specific examples of species of analytes are selected from CD markers, such as CD3, CD4, CD8, CD16, CD19, CD22, CD34, CD45, CD61, and CD91, Epithelial Membrane Antigen (EMA), Estrogen Receptor α (ERα), Cytokeratin Human, Cytokeratin 7, Cytokeratin 20, Ki-67/PI, Phosphatidylserine, BCL2 Oncoprotein, suPAR (soluble urokinase Plasminogen Acivator Receptor), urokinase, a hormone bound to a receptor, a cell cycle related protein, a marker of apoptosis, and Green fluorescent protein (GFP). These examples of analytes are all proteins that can be targeted using antibodies. The majority of these have the further advantage that monoclonal antibodies directed against their epitopes are commercially available.

According to one embodiment of the invention, the species of analyte is a receptor with a bound hormone such as a hormone bound to interleukine. When a hormone is bound to a receptor the latter often changes its conformation so that other epitopes are exposed and such that an antibody can distinguish between an unbound receptor and a receptor with a bound hormone.

Further examples of species of analyte are nucleotide sequences. Examples of such species of analyte include a chromosomal DNA sequence, a mitochondrial DNA sequence, a chloroplast DNA sequence, a mRNA sequence, a rRNA sequence, a nucleotide sequence comprising a single nucleotide polymorphism. mRNA and rRNA sequences are present in relatively high numbers whereas specific protein coding chromosomal nucleotide sequences are present fewer copies. The total number of mRNA molecules in a typical viable mammalian cell varies from 1x10⁵ to 1x10⁶ depending on tissue type and conditions. The number of specific mRNA molecules in a typical human cell varies from about 10 copies per cell (low abundance) to about 10,000 copies per cell (high abundance). The number of active genes in a typical mammalian cell is approximately 1.5x10⁴ covering less than 3% of the human genome, which is 3.3x10⁹ base pairs.

One further particular group of species of analyte are medical markers, which are markers of a disease. Assessing properties related to cells on the basis of medical markers can be used in diagnosis of various diseases and in evaluation of the clinical efficiency of drug candidates.

The disease may be selected from cancer, genetic diseases, heart diseases, infectious diseases, immune-related diseases. More specifically the disease may be selected from breast cancer (e.g. Human Estrogen Receptor 2 marker), ovarian cancer (e.g. ovarian carcinoma antigen CA125 marker), epithelial carcinoma (Epithelial Membrane Antigen marker), malignant melanoma (S-100 protein marker and CDK4 marker), leukemia (Common acute lymphocytic leukimia antigen or CD10 marker), sepsis (C-reactive protein or Caltitonin). The number of markers per cell is between 10³ to 10⁶ depending on the type of marker and the disease conditions.

A further group of diseases which can be assessed using the present invention are infectious diseases. When assessing viral diseases the particle is selected from vira such as HIV, Hepatitis virus, Herpes virus, Epstein-Barr virus, Human Papilloma virus, chlamydia virus, cytomegalovirus, influenza virus. These viruses can be assessed by using either a species of analyte, which is an antigen located in a cell infected by said virus, or by using a species of analyte located in or on the virus, said species of analyte a nucleotide sequence, or a coat protein. The number of virus copies per millilitre (viral load) is between 10² to 2x10⁶ depending on the virus type and the disease conditions. This roughly corresponds to 1 to 1,000 copies per cell depending on the virus type and the disease conditions.

### Targeting species

Different types of targeting species may be used in accordance with the present invention, two important types being antibodies and nucleotide probes. Other types of targeting species include ligands or other known binding partners.

When the targeting species is an antibody this is directed to the analyte species. Examples of antibodies include monoclonal antibodies, polyclonal antibodies and/or chimeric or synthetic antibodies. Also functional fragments of antibodies are envisaged.

The targeting species may also be a nucleotide probe complementary to a sequence of an analyte species. The nucleotide probe may comprise monomers selected from the group comprising DNA, RNA, PNA, LNA and modified analogues thereof. It is generally known that probes comprising one or more LNA monomers in the hybridising sequence have a higher Tₘ than probes with DNA, RNA or PNA monomers only.

One particular type of targeting species are In situ hybridisation (ISH) probes. Among the ISH-probes the fluorescent in situ hybridisation probes (FISH) are preferred, since they can giver higher signal/noise ratios and therefore can detect smaller nucleotide sequences without amplification.

### Selective binding

The term selective binding is used synonymously with the term selective linkage, i.e. a linkage that is specific for the analyte the parameter of which is to be assessed. In one embodiment the selective linkage is antigen-antibody binding, using antibodies, such as monoclonal antibodies, directed to an epitope on the analyte. In another embodiment the selective linkage is between nucleotide sequences.

The antibodies and/or nucleotide probes may be labelled directly or indirectly. Direct labels typically include phycoerythrin, (RPE or PE), cyanine dyes (Cy dyes), Cy3, Cy5, Cy5.5, Cy7, allophycocyanines (APC), indotrimethinecyanines, indopentamethinecyanines, acridine orange, thiazole orange, DAPI, propidium iodide (PI), ethidium iodide, 7-aminoactinomycin D, Per CP or chemically coupled combinations thereof. Indirect labels include secondary antibodies against the primary antibody, said secondary antibodies being labelled with a labelling agent.

In an indirect label the primary antibody or nucleotide probe directed against an epitope or nucleotide sequence on the analyte may be linked covalently to a compound such as biotin, streptavidin, avidin, a hapten, digoxigenin, dinitrophenyl or fluorescein. The analyte may then be visualised by a second label which specifically binds to the compound linked to the primary antibody or probe. Examples of such indirect labels include but are not limited to: hapten anti-hapten complex; biotin streptavidin complex; biotin avidin complex; digoxigenin anti-digoxigenin complex; dinitrophenyl anti-dinitrophenyl complex; fluorescein anti-fluorescein complex.

In the case where the primary antibody or probe is linked to biotin, signal amplification can be obtained by linking streptavidin to the biotin and further linking biotin-label-biotin complexes to the streptavidin. Further rounds of amplification can be obtained by linking further streptavidin and biotin-label-biotin complexes. The result is that several labels are linked via complex linkages to the epitope or nucleotide sequence instead of just one label.

In another embodiment the species-selective linkage may be provided using DNA and/or RNA and/or PNA and/or LNA probes selective for DNA and/or RNA related to the analyte. The probes may be labelled directly or indirectly through additional probes as described above in relation to labelling of antibodies. According to one embodiment, branched DNA (bDNA) can be used for signal amplification. bDNA involves the use of nucleotide amplifiers, label probes, label extenders and optionally capture extenders such as described in US 5,635,352 and 5,124,246 (Chiron).

The invention also comprises the feature that an additional linkage is formed between a second species of analyte and a second label.

In the following non-exclusive list of examples of selective linkages formed between the analyte and the label, an asterisk * denotes an affinity binding or a nucleotide hybridisation such as the binding between antibody and antigen or the binding between avidin and biotin or the binding between homologous nucleotide sequences. A dash - denotes a covalent bond.
analyte*antibody-label
   analyte*antibody*antibody-label
   analyte*antibody-biotin*avidin-label
   analyte*antibody-biotin*streptavidin-label
   analyte*antibody-avidin*biotin-label
   analyte*antibody-streptavidin*biotin-label
   analyte*antibody*antibody-avidin*biotin-label
   analyte*antibody*antibody-streptavidin*biotin-label
   analyte*antibody*antibody-avidin*biotin-label
   analyte*antibody* antibody-streptavidin*biotin-label
   analyte*antibody-biotin*streptavidin*(biotin-label-biotin)ₙ*streptavidinₙ etc
   analyte*antibody-digoxigenin*antidigoxigenin-label
   analyte*antibody-dinitrophenyl*antidinitrophenyl-label
   analyte*antibody-hapten*antihapten-label
   analyte*antibody-fluorescein*antifluorescein-label
analyte*DNAfragment-label
   analyte*DNAfragment*DNAfragment-label
   analyte*RNAfragment-label
   analyte*RNAfragment*RNAfragment-label
   analyte*PNAfragment-label
   analyte*PNAfragment*PNAfragment-label
   analyte*LNAfragment-label
   analyte*LNAfragment*LNAfragment-label
   analyte*DNAfragment*RNAfragment-label
   analyte*DNAfragment*LNAfragment-label
   analyte*DNAfragment*PNAfragment-label
   analyte*RNAfragment*LNAfragment-label
   analyte*RNAfragment*PNAfragment-label
   analyte*RNAfragment*LNAfragment-label
   analyte*nucleotide probe-biotin*streptavidin*(biotin-label-biotin)ₙ*streptavidinₙ etc
   analyte*nucleotide probe-digoxigenin*antidigoxigenin-label
   analyte*nucleotide probe-dinitrophenyl*antidinitrophenyl-label
   analyte*nucleotide probe-hapten*antihapten-label
   analyte*nucleotide probe-fluorescein*antifluorescein-label
   analyte*bDNA probe*label probes

It is likewise conceivable that the species specific linkage may be formed by reversing the position of two components participating in the affinity binding such as reversing the order of avidin*biotin or RNA*PNA etc.

### Sample

The sample may be a liquid sample such as a sample selected from the group consisting of milk, milk products, urine, blood, sperm, nasal secrete, tears, faeces, waste water, process water, drinking water, cerebro-spinal fluid, gall, bone marrow, food, feed, and mixtures, dilutions, or extracts thereof.

The method may also relate to assessment of particles in water, such as control of drinking water, control of waste water or water from a water purifying plant or swimming pool. In all applications the control may be related to the total particle count, such as bacteria count or it may more particularly be related to a monitoring process for specific bacteria, such as pathological bacteria.

Furthermore, fermentation control, i.e. control of cell growth and viable cells in fermentation tanks may be conducted by the invention. This relates to all technical and industrial fields using fermentation, such as the pharmaceutical industry for producing peptide or protein compositions.

The liquid sample may be pre-treated with any suitable treatment, such as centrifugation, sedimentation, filtration, extraction, dilution, irradiation, agitation, addition of chemicals, chromatographic separation.

In another embodiment the sample is a solid sample which is pre-treated prior to being arranged in the sample domain. Examples of pre-treatments are blending, homogenisation, (re)suspension and dissolution. A pre-treatment is optionally followed by any of the treatments mentioned for the liquid sample.

The sample may be any biological sample, such as a biopsy of tissue, such as a biopsy of muscle, brain, kidney, liver or spleen.

Also, the sample may be a sample of food or feed to be tested for contamination, such as bacterial contamination. The present invention offers a very fast method of detecting and enumerating bacteria in food or feed such as a method of detecting Salmonella species. The sample may also be a soil sample.

Independent of the form of sample it is required that the particle is suspended in a medium before contacting the substrate. Said medium may be the natural medium for the particle or any liquid suitable for the detection. In one embodiment the particle is suspended in a medium after being pre-treated.

### Fluorescence

In a preferred embodiment the label is a fluorescent compound. A system based on fluorescence is generally more sensitive than a chromogenic since fewer product molecules are necessary for providing enough electromagnetic radiation to visualise the particles.

A fluorescent label is preferably capable of emitting signals in the wave length range of from 300 to 1200 nm when excited by excitation light. One preferred fluorescence method is the method of polarised fluorescence.

The excitation light source is any suitable light source capable of emitting excitation light in the range of from 250 nm to 800 nm, such as a light emitting diode (LED), a gas laser, a solid state laser, a laser diode, a gas lamp, a halogen lamp, or a xenon lamp.

It is preferred to use a diverging excitation light, such as light emitting diodes for in a cost-effective manner to expose as large area as possible of the sample to the excitation light.

It may be preferred to use more than one light source for the purpose of increasing the flux of light onto the sample, for instance by using two or more light emitting diodes. It is also possible to use more than one light source where some of the light sources have different electromagnetic properties.

By the use of several LEDs the sample is exposed to excitation light from several angles leading to a substantially optimal excitation of the sample, the light sources are preferably operated in such a way that all transmit substantially simultaneously.

However for some applications wherein at least a first and a second light source are arranged in the first excitation light means, the first light source having a different wavelength band than the second light source, the light sources may transmit in an alternating manner. By the use of two different light sources it is possible to obtain two different fluorescence signals from the sample. There is no upper limit to the number of LEDs used, but often as many as 30 LEDs are provided, such as up to 50 LEDs, for example up to 100 LEDs, such as up to 150 LEDs, for example up to 200 LEDs, such as 300 or more LEDs.

If a less diverging light source is used a diverging optical means may be arranged in the excitation light path to diverge the excitation light properly.

When using laser diodes as the excitation light the proper divergence may be accomplished by an arrangement of at least 4 laser diodes optionally provided with diverging means.

The incident angle of the excitation light is preferably in the range between 0° and 90°, to the optical axis of the detection system, more preferably between 0° and 60°, such as between 10° and 45° to provide a suitable excitation of the sample.

The excitation light may be transmitted directly to the sample, i.e. without being deflected by a beam splitter or the like whereby it is possible to construct the system and apparatus more compact.

### Dual or multiple colour

In a preferred embodiment, a distinction between at least two spectral properties of labels is used in the process of obtaining at least one quality parameter or at least one quantity parameter of the particles. Thus, by the present invention it is possible to simultaneously detect at least two different types of particles. This is achieved by using at least two antibodies or two nucleotide probes directed towards two different analytes located in or on two different types of particles, and providing the two antibodies or probes with two different labels, either directly or indirectly, and detecting the two different particles.

The two different types of particle may be two different cells, for example specific IgG and IgA-secreting cells, or the two different states of the same cell, such as to distinguish between dead and living cells, or distinguishing between apoptotic and non-apoptotic cells, or distinguishing between cells having different membrane antigens exposed on the surface, e.g. different CD marker. It may e.g. be of interest to be able to distinguish T cells from B cells in one and the same sample. This can be done by using a CD3 marker as the species of analyte for T-cells and a CD19 marker as the species of analyte for B-cells. Phosphatidylserine is present on the surface of early apoptotic cells and may be detected with fluorescently labelled Annexin V in order to distinguish apoptotic cells from non-apoptotic cells.

Also, in the latter situation a dual labelling may be carried out by using one labelled antibody directed towards the analyte and then another type of labelling of the particles to distinguish dead from living cells, such as conventional viability staining.

It is understood, that more than two different particles may be assessed hereby, such as three particles for example.

### Sample domain

The sample domain established according to the present invention may be a compartment or an equivalent thereof, wherein the sample is located during recording, such as a three-dimensional sample domain.

The sample domain may be a part of a flow-through system, wherein each sample is part of a series of samples, whereby one sample is replacing the previous sample in the sample domain. In such embodiments, the sample compartment has both an inlet and an outlet. In other embodiments, the sample compartment only has an inlet.

In one particular embodiment the sample domain is part of a cassette, such as a disposable cassette as described in PCT/DK99/00605. In some embodiments, such a cassette contains pre-added chemicals that contribute to generation of the signal.

The sample is contained in the interior of the sample compartment, which has an average thickness of between 20 *µ*m and 200 *µ*m, usually between 30 *µ*m and 150 *µ*m and in many practical embodiments between 50 *µ*m and 100 *µ*m.

The part of the sample domain allowing signals to be detected is referred to as the exposing window that can be as little as 1 mm² or more, preferably with an area of 2 mm² or more, preferably with an area of 4 mm² or more, preferably with an area of 10 mm² or more, preferably with an area of 20 mm² or more, preferably with an area of 40 mm² or more, more preferably with an area of 100 mm² or more.

### Sample volume

Sample volumes as small as 1 ml or less and even as small as 0.02 ml can be used. The optimal volume of the sample needed is highly dependent on the number of particles present in the sample and the predetermined statistical quality parameter sought.

Other preferred embodiments of the present invention make it possible to assess particles from a considerably large volume of sample. This can allow the measurement of samples with only few particles of interest per volume of sample. Sample volumes larger than 1 ml and even larger than 100 ml can be used for the analysis, the volume being defined as the total volume of any liquid sample introduced to any flow system connected to the device before the measurement of the sample.

Often the design of the sample compartment or the sample is such that the size of the volume of the liquid sample is sufficiently large to permit the assessment of the at least one quantity parameter or the at least one quality parameter to fulfil a predetermined requirement to the statistical quality of the assessment based on substantially one exposure, so that the image is recorded in one exposure. In another embodiment the assessment of at least one quality parameter or at least one quantity parameter is obtained on the basis of more than one image, preferably two images, more preferably more than two images, more preferably more than four images. In these situations the images are recorded through two, three or more exposures. This can for instance be done to fulfil a predetermined requirement to the statistical quality.

Also, information about the changes in the image in course of time is used in the assessment of at least one quality parameter or at least one quantity parameter, and in such situations more than one exposure is made.

In many assessments of particles it is of interest to allow exposure of signals from substantially large volumes of sample. The volume of the liquid sample from which signals such as electromagnetic radiation is exposed onto the detection system is normally in the range between 0.01 *µ*l and 20 *µ*l. Generally the volume of the sample being analysed should be as large as possible. This allows the simultaneous assessment of a higher number of particles, but the optimal volume is often defined by one or more aspects of the detection system and the sample being analysed. Thus the volume of the sample in the sample compartment can be less than 0.1 *µ*l but often a volume of more than 0.1 *µ*l, more than 1.0 *µ*l or even more than 10 *µ*l is used. In still other applications, a volume of the sample compartment as large as 100 *µ*l or more can be used.

A large volume of the sample is preferably measured by passing the volume of sample through a particle retaining means, such as a filter, electrical field, magnetic field, gravitational field, such means preferably being included in the device or can be arranged to interact with any sample within the device. The particle retaining means should preferably be able to retain substantially all particles present in a sample, or at least a substantially representative fraction of at least one type of particle present in the sample.

When the particles from a large sample are retained, those particles can be resuspended in a volume which is less than the volume of sample passed through the particle retaining means.

In one embodiment more than one portion of the same sample material can be subjected to analysis by exposure to the detection system. This can be done by allowing the sample compartment to be moved, thus exposing a different portion of the sample compartment.

### Magnification

The method is preferably carried out at a low magnification whereby it is possible to detect particles in a large volume in one or a few exposures. The magnification factor is below 20, such as below 10, such as below 5, such as 4, more preferably below 4, such as 2, more preferably below 2, such as 1. The advantages of such low magnification are several, among other things increased area of observation and increased depth of focusing implying increased volume exposed to the detection device.

When the particles in question have dimensions which are comparable to the size of a detection element, it is often preferred to have magnification of about 1/1, thus focusing the image of any particle on any one or just few detection elements. This can under some condition give favourable detection of any signal.

When analysing particles which have dimensions which are comparable to, or bigger than the detection elements used, it is often advantageous to reduce the size of the image of such particle, to a degree where the size of the image is comparable to the size of a detection element. Thus in one embodiment it is preferred that the magnification factor below 1, preferably below 0.9, such as 0.8, more preferably below 0.8 such as 0.6, more preferably below 0.6 such as 0.5.

In these situations it is preferred that the ratio of the size of a particle, to the size of the image of the particle on the array of detection elements is 1/1 or less, preferably less than 1/1 and higher than 1/100, more preferably less than 1/1 and higher than 1/40, more preferably less than 1/1 and higher than 1/10, more preferably less than 1/1 and higher than 1/4, more preferably less than 1/1 and higher than 1/2.

Thus, it is often preferred that the spatial representation exposed onto the array of detection elements is subject to such a linear enlargement that the ratio of the image of a linear dimension on the array of detection elements to the original linear dimension in the sample domain is at the most 20:1, preferably smaller than 10:1 and in many cases even at the most 6:1 or even smaller than 4:1.

It is often preferred that the image of the product from the individual particles the parameter or parameters of which is/are to be assessed are imaged on at the most 25 detection elements, in particular on at the most 16 detection elements and more preferred at the most 9 detection elements. It is even more preferred that the image of the product from the individual particles the parameter or parameters of which is/are to be assessed are imaged on at the most 5 detection elements, or even on at the most 1 detection element. The larger number of elements per particle will provide more information on the individual particles, while the smaller number of elements per particle will increase the total count that can be made in an exposure.

### Statistics

As mentioned above, the size of the volume is suitably adapted to the desired statistical quality of the determination. Thus, where the determination is the determination of the number of particles in a volume, or the determination of the size and/or shape of particles, the size of the volume of the liquid sample is preferably sufficiently large to allow identification therein of at least two of the particles. More preferably, the size of the volume of the liquid sample is sufficiently large to allow identification therein of at least four of the particles. This will correspond to a repeatability error of approximately 50%. Still more preferably, the size of the volume of the liquid sample is sufficiently large to allow identification therein of at least 10 of the particles. This will correspond to a repeatability error of approximately 33%. Even more preferably, the size of the volume of the liquid sample is sufficiently large to allow identification therein of at least 50 of the particles. This will correspond to a repeatability error of approximately 14%. Evidently, where possible, it is preferred to aim at conditions where the size of the volume allows identification of even higher numbers. Thus, when the size of the volume of the liquid sample is sufficiently large to allow identification therein of at least 100 of the particles, it will correspond to a repeatability error of approximately 10%, and when the size of the volume of the liquid sample is sufficiently large to allow identification therein of at least 1000 of the particles, it will correspond to a repeatability error of as low as approximately 3%.

### Stand still

According to the invention the particles being assessed are at stand still or substantially at stand-still during analysis, thus allowing the optimal use of measurement time in order to improve any signal to noise conditions. This arrangement also eliminates any error which could be inherent in the assessment of particles caused by variation in flow conditions, particularly when an assessment of a property is a volume related property such as the counting of particles in a volume of sample.

### Flow system

The introduction of particle and reagent material into the sample domain may be provided by means of a flow system. The flow system may provide at least one of several operations to be carried out on the samples, said operations being selected from but not limited to transport, mixing with reagent, homogenising of sample and optionally reagent, heat treatment, cooling, sound treatment, ultra sound treatment, light treatment and filtering.

In order to flow the sample into or within or out of the sample domain it is preferred to have at least one propelling means provided in the system.

Preferably the flow regulation means is arranged to function stepwise so that the sample and/or the reagent component may be flowed stepwise through the system.

The sample in the device can be flown by the means of a flow system, which can be driven by a pump or a pressurised gas, preferably air, or by causing a pressure difference such that the pressure on the exterior of the inlet is higher than the pressure within at least a part of the system thus forcing the sample to flow through the inlet. In many embodiments of the present invention the flow in said flow system is controlled by one or more valves which can adjust the flow speed of the sample.

In many preferred situations the flow of liquid in the device can be brought about by a vacuum, the vacuum being applied from a reservoir, preferably contained within the device. The vacuum can be established by a mechanical or physical action creating the vacuum substantially simultaneously with the introduction or the movement of the sample. These mechanical or physical actions can be: a peristaltic pump, a piston pump, a membrane pump, a centrifugal pump and a hypodermic syringe.

The outlet from the sample compartment can be passed through a flow controlling means, such as a valve, which only allows gas to pass through. One such type of valves which often is preferred, is one which allows gas and air to pass but can close irreversibly when the valve comes in contact with liquid sample. The effect of such valve is to minimise the movement of any sample within the sample compartment during analysis.

In a preferred embodiment of the invention the system contains at least one compartment wherein the mixing of the sample material with catalyst and/or media is possible.

One advantage of the present system and method is that the analysis is carried out using only liquid reagents and particles suspended or dissolved in liquid. This layout ensures ease of operation and handling.

### Detection device

The image which can be detected from the window of the device can for instance be detected by an array of detection elements, the array of detection elements comprising individual elements, each of which is capable of sensing signals from a part of the sample window area, the array as a whole being capable of sensing signals from substantially all of the sample window area, or at least a well defined part of the sample window area. The array of detection devices may for example be a one-dimensional array or a two-dimensional array. In order to facilitate the assessment of particles the intensities detected by the array of detection elements are processed in such a manner that representations of electromagnetic signals from the particles are identified as distinct from representations of electromagnetic background signals.

The detection means may comprise any detectors capable of sensing or detecting the signal emitted from the sample such as a fluorescence signal.

According to the invention detection means comprise a detector being an array of detecting devices or detection elements, such as a charge coupled device (CCD) the CCD may be a full frame CCD, frame transfer CCD, interline transfer CCD, line scan CCD, an eg. wavelength intensified CCD array, a focal plane array, a photodiode array or a photodetector array, such as a CMOS. The CMOS is preferably a CMOS image sensor with on-chip integrated signal condition and/or signal processing. Independent of the choice of any of the above detection devices the detection means may further comprise a white/black or colour CCD or CMOS.

Confocal scanning optical microscopes are known in the art and offer a number of advantages over traditional optical microscopes. One main advantage of a confocal scanning microscope is that it provides optical sectioning of a sample because it attenuates light which is not in focus. Thus, only light which is in focus contributes to the final image.

In a scanning confocal microscope, a beam is swept across a surface of a sample. The light which emanates from the sample (e.g., reflected from, emitted from or transmitted through) is directed towards a pinhole. Light that is in focus passes through the pinhole and onto an optical detector. As the beam is scanned across the surface of the sample, the output from the optical detector can be accumulated and formed into an image of the scanned surface.

Use of a confocal scanning microscope especially a confocal laser scanning microscope for detecting the signals from the product formed in the sample domain is advantageous due to the greater sharpness of the detected image.

The size of the detection elements determines to some extend its sensitivity. In some applications it is therefore of interest to have detection elements of size of about 1 *µ*m² or less. In certain situations the size of the detection elements in the array of detection elements is less than 20 *µ*m², preferably less than 10 *µ*m², more preferably less than 5 *µ*m², more preferably less than 2 *µ*m², more preferably less than or equal to 1 *µ*m².

The array of detection elements is preferably sensitive to electromagnetic radiation of wavelength in one or several of the following regions: 100 nm to 200 nm, 200 nm to 800 nm, 800 nm to 2 *µ*m, 2 *µ*m to 10 *µ*m, 5 *µ*m to 10 *µ*m, 10 *µ*m to 20 *µ*m, 20 *µ*m to 40 *µ*m.

The inclusion of a focusing device for the focusing of a signal from the sample onto the detection elements in such a manner as to maximise the collection angle, the collection angle being defined as the full plane angle within which a signal is detected, has in many situations been found to give improved conditions for an assessment.

### Focusing - Lenses

Signals from at least a portion of the sample are focused onto the array of detection elements, by the use of a focusing means, preferably by the use of one lens, it is however possible to use two lenses, or more than two lenses. The number of lenses used for the focusing system can affect the complexity of any measuring system.

The focusing of a signal from the sample onto any detector is dependent on the position of the sample relative to any detector. When the construction of measuring system is such, that the relative position of the sample and any detector can vary, then there is advantage in being able to adjust the focusing of the system. This can often be achieved by first taking at least one measurement of any signal from the sample and then on the bases of this, to adjust the focusing of the system. This procedure can be repeated a number of times in order to obtain acceptable focusing. In the same manner the focusing of signal from the sample or sample material is adjusted, preferably where the extend of the adjustment is determined by at least one measurement of a signal from the sample.

A property of several preferred embodiments of the present invention, is that it is possible to maintain identical focusing throughout the working time of an instrument applying the invention. In preferred embodiments the elements comprising the optical system are therefore securely fixed during production, preferably they are irreversibly fixed, e.g. through gluing of the elements of the optical system, thus preventing any intentional or accidental changing of the alignment of the optical elements.

The collection angle of a focusing arrangement used can have effect on the intensity of any signal collected on the array of detection elements. When high sensitivity is needed it is therefore practical to increase the collection angle. The preferred size of the collection angle can also be determined by other requirements which are made to the system, such as focusing depth. In these situations the collection angle of the focusing means is preferably at least 2 degrees, preferably more than 5 degrees, more preferably more then 15 degrees, more preferably more than 20 degrees, more preferably more than 50 degrees, more preferably more than 120 degrees, more preferably more than 150 degrees.

### Signal

The signals measured from one or more detection elements may be corrected for systematic or varying bias by the use of a calculating means, the bias correction being accomplished by the use of one or more pre-defined value(s), preferably where each measured signal for one or more detection elements in said array of detection elements has one or more pre-defined value(s), more preferably where each pre-defined value is determined on the bases of one or more of any previous measurements.

The bias correction may be performed by subtracting the results obtained in one or several of other measurements from the measured signal, preferably where the other measurements are one or several of measurements of the same sample, or sample material, more preferably where the other measurement is the measurement taken previously of the same sample or sample material.

Also the signal from one or more detection elements may be corrected for intensity by the use of a calculating means, said correction being accomplished by the use of one or more pre-defined value(s), preferably where each measured signal for one or more detection elements in said array of detection elements has one or more pre-defined value(s), more preferably where each pre-defined value is determined on the basis of one or more of any previous measurements.

### Processor

Information of the signals detected by the detection means are input into a processor for processing, displaying and optionally storing the information.

The at least one quality or at least one quantity parameter of the particles is obtained by processing of the signals detected by the detection means. This processing can e.g. include conversion of the raw data using a pre-determined algorithm to obtained the quality or quantity parameter. The processing can also include use of a calibration curve or standard curve that specifies the relationship between the signal and the parameter of interest, e.g. as described in the Examples.

The signal information may be displayed on a display connected to the processor and/or printed. The information displayed may be any kind of information relating to the signals measured and/or the system used, such as a number, size distribution, morphology, classification of particles, excitation wavelength, emission wavelength, magnification. In particular the data processing means is capable of distinguishing partially overlapping areas of product.

It is possible to make the processing and the assessment of the parameters of the sample by using a calculation mean, preferably a digital computer, one commercially available from Analogue Devices (ADSP 2101), equipped with storage capacity which can only store information in amount substantially equivalent to a small fraction of the total number of detection elements, the assessment of the number of objects then being based on substantially real time processing of data, preferably in such a way that the measured information from each detection element, or a line of detection elements, or two or more lines of detection elements, is used for the assessment, substantially without any delay, such as a delay which would otherwise be caused by storing the measured information.

However, it is often preferred to store substantially all measured information by the use of a first calculation mean, preferably a digital computer, before the processing of the information by a second calculation mean, preferably a digital computer, and thus allowing the measured information to be processed at substantially the same rate it is obtained, but with a substantial time delay between the measurement of any information and the processing of the same information; preferably, this is accomplished by using only one calculating mean, preferably a digital computer, equipped with enough resources to accomplish the task.

### One-sided and two-sided systems

One-sided and two-sided systems for the detection of particles are known from WO 01/77648 (Chemometec).

The detection device may be laid out as a one-sided device, i.e. a device for which the light is directed to the sample from the same side of the sample as the side from which the signals are detected.

The detection device may also be laid out as a one-sided device, in which the excitation light is directed to the sample from the same side of the sample as the side from which the signals emitted from the sample are detected.

By this apparatus a variety of advantages have been achieved as compared to conventional fluorescence microscopes. First of all it is possible to arrange the sample to be assessed directly in the sample plane instead of sliding it into the sample plane between the detector and the excitation light. Furthermore it has become possible to detect surface fluorescence of a sample not being transparent. It is also possible to increase the intensity of the excitation light without compromising the detectors.

Also samples having a nature whereby it is normally not possible to arrange the sample in a microscope may be assessed by the use of the present system, in that the microscope may be placed directly on the sample whereby the surface of the sample simply constitutes the sample plane.

Finally it is possible to produce a more compact and thereby more easily handled apparatus, in that the excitation light means is arranged on the same side of the sample plane as the detector, thus shortening the axis of the apparatus by at least 25% as compared to conventional apparatuses.

By the present invention it is possible to assess parameters of a sample which has up to now only been reliably assessed by the use of flow cytometric equipment. It is possible to assess parameters of a large sample in one exposure thus reducing the statistical errors normally counted for when assessing large samples by assessing only parts thereof per exposure.

Furthermore, it is possible to obtain more than one fluorescence signal from the sample in one exposure thereby facilitating classification of particles of the sample, due to their different fluorescence signals.

### Examples of one and double sided excitation and detection systems

In the following one embodiment of the detection system is discussed in more detail in relation to the drawings.

In Fig. 18 an example of the illumination and detection system 1 is shown in schematic form. The sample is arranged in a sample compartment in the sample plane. Excitation light from the light sources 4a, 4b in the excitation light means 3 is exposed onto the sample through a main light path 5a, 5b.

Fluorescence signals from the sample is emitted to the detection means 6 comprising at least one detector 7. The path of the emitted signals is following an axis between the sample and the detector, the detection-sample axis 8.

The signal data are transmitted to a processor coupled to the detecting means 6. The fluorescence signals from the sample is filtered by means of emission filter 14 and focused to the detection means 9 by means of a focusing lens 10.

The light sources 4a, 4b are arranged in a light module 11, whereby the transmission of excitation light directly to the detection means is avoided thereby increasing the system's relative signal to noise ratio. Furthermore excitation light filters 12a, 12b are positioned in the excitation light beam.

Fig. 19 shows a cross-section of the circular supporting material 13 of the excitation light filters wherein the position of the light sources have been indicated by circles in broken lines.

In Fig. 20 the light path and signal path is shown in more detail. In the light path the main light path is shown as 5. Furthermore, the detection-sample axis is shown by broken lines 8. The collection angle of the system is denoted C shown between two arrows and the angle between the main light path and the detection-sample axis is denoted E.

In Fig. 21 a double-sided excitation/detection system 1 is shown wherein the systems on each side of the sample are identical and as described for the one-sided system of Fig. 1.

Fig. 22 shows a double-sided excitation system wherein excitation light from the light sources 4a, 4b in the first excitation light means 3a and excitation light from the light sources 4c, 4d in the second excitation light means 3b is exposed onto the sample 2 from both sides of the sample 2. As discussed above, the light sources may be identical or different depending on the information to be assessed. Furthermore, the filters used for each light source may be different or identical.

Fluorescence signals are transmitted through and reflected from the sample due to the excitation light arrangement and emitted to the detection means 6. The path of the emitted signals is following an axis between the sample and the detector, the detection-sample axis 8.

The signal data are transmitted to a processor coupled to the detecting means as described above.

Fig. 23 shows a double-sided detecting system, using a single-sided excitation system, wherein reflected fluorescence signals from the sample 2 are detected by detecting means 6a comprising detector 7a. The reflected fluorescence signals are transmitted though filter 14a and focused by lens 10 a.

Furthermore, transmitted fluorescence signals from the sample 2 are detected by detecting means 6b comprising detector 7b. The reflected fluorescence signals are transmitted though filter 14b and focused by lens 10b.

Filter 14a is preferably different from filter 14b, whereby information relating to at least two different fluorescence signals is obtainable.

Also the magnification in the two detecting systems may be different, for example by lens 10a being different from lens 10b.

Fig. 24 shows one preferred construction of a imaging system, where up to 4 interchangeable emission filters 14 are placed on a wheel, which can be rotated by a motor 140. The excitation light module 11 of this construction comprises up to 102 light emitting diodes (LED's), which can be of the same type, or of different types, activated independently. The light from the LED's is focused onto the sample 2 by a fresnel lens (Frenel Optics, Germany, Cat.No. 02560001, SC256 FL=25.4mm, positive aspheric lens). The combination of more than one wavelength of excitation light and more than one emission wavelength make this construction suitable for the detection of two or more analytes, by a method of exposing sequential images.

### Example 1

### Experimental study on detection and quantification of specific surface markers on human blood cells.

### Procedure

A calibration curve was constructed by measuring beads with known fluorescence intensities (QuantibritePE beads, Becton-Dickinson, Cat.No. 340495) diluted in an IgG1/phycoerythrin (PE) stained control blood sample. It was possible to detect and distinguish three of the four populations of fluorescent beads based on figure 7 to 10, where there are three distinct populations of beads above the background level (see also Table 2 with data). Two different lots of beads were measured in order to distinguish at least four different populations of beads. This enabled the construction of a calibration curve that specifies the correlation between the integrated fluorescence intensity (IFI) and the number of fluorescent PE molecules on the individual objects being measured (known from the manufacturer, Becton-Dickinson QuantibritePE beads CAT# 340495). The calibration curve is of course specific for the electronic settings and for the chemical procedure used. Two different lots of QuantiBritePE beads (Becton-Dickinson QuantibritePE beads CAT# 340495) were used in order to obtain more points to the calibration curve. Lot. 20977 and lot. 32417 were used. The beads have diameter <10µm.

### Bead Calibration Curve in PBS:

BD QuantiBrite PE beads Cat.No. 340495 in PBS buffer with 0.5% Pluronic P3100 (defoaming reagent, BASF Performance Chemicals Division) prior to analysis.

### Bead Calibration Curve in IgG1/PE control sample:

QuantiBritePE beads were dissolved in IgG1/PE stained control blood sample prior to analysis (see below). Blood sample was pre-diluted 1:4 in PBS buffer with 0.5% Pluronic P3100 (defoaming reagent, BASF Performance Chemicals Division) prior to dissolving the beads.

A common commercial "no wash" procedure for flow cytometry analysis was used for staining cellular surface markers on human peripheral blood cells (whole blood, EDTA anti-coagulated). The staining procedure requires no centrifugation or washing of the cells prior to analysis (DAKO Uti-lyse S-3350, "no-wash" protocol, DAKO A/S, Denmark).

Antibodies were anti-CD3/PE (Sigma P-5810), anti-CD4/PE (Sigma P-7562), anti-CD8/PE (Sigma P-5560), anti-CD45/PE (Sigma P-7687), Mouse isotype negative controls IgG1/PE (Sigma P-4685), IgG2a/PE (Sigma P-4810). The protocol was performed as described by the manufacturers of the reagents. (DAKO Uti-Lyse cat.no. S-3350). Samples were diluted up to 4 times in PBS buffer with 0.5% Pluronic PE3100 (defoaming reagent, BASF Performance Chemicals Division) prior to analysis.

Exposure time was 2 secs for all images (except for CD4 where exposure time was 3 secs), and transversal magnification is close to 1 (M_{T}=0.98). The time for capturing one image, processing and saving data, and moving the liquid through the cuvette is approx. 5 sec. A total of 20-40 images were captured in about 100-200 sec.

### "No Wash" staining procedure:

1. Add 100µL of anticoagulated (EDTA) whole blood
2. Add 10µL of the conjugated antibody specified to the blood, vortex and incubate for 30minutes.
3. Add 100µL of Reagent A (fixative reagent), vortex and incubate for 10 minutes.
4. Add 1 mL of Reagent B (lysing reagent), vortex and incubate for 15 minutes.
5. Add 3 mL of PBS buffer with 0.5% Pluronic PE3100 (defoaming reagent) to dilute approx. 1:4 .
6. Analyse the sample on the ChemoMetec fluorescense detection and imaging system.

Total dilution from whole blood is then approx. 38 times when the above "no wash" protocol is used.

The sample is analysed by injecting it into the sample cuvette using a 1 mL syringe (se picture in Figure 3). The fluid is stopped before capturing images by attaching a tubing squeeze-stopper to the tubing leading out from the cuvette (not shown).

### Experimental setup

The picture in Figure 1 shows the modified microscopic module originally from the NucleoCounter ChemoMetec product. A diagram of the experimental system is shown in Figure 2. The lenses and filters have been modified. The modified module has increased numeric aperture (NA= approx 0.10) but still low transversal magnification (M_{T}= approx. 0.98).

### Settings and specs:

Object size discrimination: 6 pixels (incl.)
Cell counting threshold = 10 IFI (generally background objects had IFI<10)
Raw image background, median filter maximum (width=41): 10
Exposure time: 2sec ("D19"= 7, except CD4 where expose time=3 sec)
Exposure time code: Expose = 0.266 x (1 + "D19")
Settings: gain=85, offset=120, range=200 (except CD3 where gain=90 and CD4 where D19=11)
PC type: Dell Inspiron 2500 (Laptop with Intel Pentium III, 800MHz, 128Mb RAM, with USB port)
PC software:
   Operative system: Microsoft Windows 2000 (Microsoft)
   NI LabVIEW v.6i (National Instruments, Texas USA)
   NI IMAQ vision (National Instruments, Texas USA)
   Image depth: 8 bit (256 colors greyscale)
   CCD type: SONY ICX404AL, 510x492 pixels, interlaced readout (images are 510x246 pixels)
   CCD physical size: 4.90 mm x 3.69 mm (4:3 scale)
   Excitation filters: Omega 510LAF21 (Ø=25.0 mm) and Ferroperm 2xSWP550+IRblock (Ø=25.0 mm)
   Emission filter: Ferroperm OG560+2xLWP575 (Ø=25.0 mm)
   Lens types: Edmund Scientific part no 45400. EFL = 25.0 mm
   optical system NA= approx. 0.10
   optical system M_{T}= SQRT( (4.90x3.69x 101.2)/1905)) = approx. 0.98 (close to 1)
   Dot cuvette grid specs: 101.2 dots equal 1.00 mm²

### Observations:

Mean Cell Area: approx. 2.8 to 3.1 pixels.
(Objects with high IFI had larger mean area than objects with low IFI)

Components of the experimental "double triplet system" (Figure 1) are listed in Table 1 below.

**Table 1. "Double triplet" detection system components:**

| **#** | **component** | **description** |
|---|---|---|
| 1 | LED fixture | 8 LEDs emitting light around 518nm |
| 2 | Excitation Band pass filter | OMEGA, XF1074, 510 LAF 21 |
| 3 | Excitation Short pass filter | Ferroperm, 2 x SWP550nm + IR block |
| 4 | Diffuser glasses | 2 pcs. diffusing side faces cuvette. |
| 5 | Cuvette measuring area | Measuring area 4.96 mm x 3.69 mm, spacer 35-45 µm |
| 6 | Cuvette body | Aluminum, cuvette glass type Boro silicate, 2 mm |
| 7 | Triplet lens 1 | Edmund Scientific part no 45400. EFL = 25.0 mm |
| 8 | Diaphragm | Diaphragm diameter = 5.8 mm |
| 9 | Emission Long pass filter | Ferroperm, OG560 + 2 x LWP575nm |
| 10 | Triplet lens 2 | Edmund Scientific part no 45400. EFL = 25.0 mm |
| 11 | CCD | CCD in fixture |
| 12 | Camera PBC | PCB for mounting the CCD |

### Image data processing

The image data may be processed in many different ways to obtain uniform distribution patterns for the objects being analysed.

In this experimental system the imaging and counting of objects was done in the PC with software based on LabVIEW, National Instruments. The counting of the fluorescent objects was also done during each image capturing by the ChemoMetec microscopic module (left in Figure 1). The two methods gave similar counts for the individual images, but the module did not summarise the data from multiple images as it was possible in the PC software analysis after capturing of the images. Therefore analyses involving more than one image were performed in the PC based software.

General description of the PC based software imaging operations:
1. **Large-width median filter** (width > 40pixel) was used on each dimension to eliminate image profile and enhance the S/N-ratio for the following operations
2. **Simple thresholding** operation was used in combination with
3. **Object size discrimination** (maximum number of pixels per object) for detection of the objects
4. **Integration of intensities,** calculation of each objects's integrated fluorescence intensity (IFI).

Other potential methods to be used for this system:
- **Background subtraction** with masking
- **Zone detection** for determination of distribution pattern within image,
- **Statistical variations analysis** for multiple images, (e.g. rough CV% analysis on specified range of total counts, empirical range of data) may be used.

The filtered and thresholded images made from images like Figure 5 and Figure 6 were automatically processed for identification of objects having IFI above the background (medial filter maximum value). The intensities were integrated over each of the objects and the resulting data were plotted into a histogram. Data from 20 to 40 different images were summarised corresponding to a total volume of approx. 15 µL to approx. 30 µL of sample mixture. The sample mixture was diluted up to 4 times after traditional immunostaining prior to analysis. Total dilution from whole blood is approx. 38 times when the above "no wash" protocol is used.

The processing of a single image took less than approx. 0.2 sec using PC based software.

Data from 20 to 40 different images were summarised and plotted into histograms from which the different populations geometric mean IFI could be estimated using manual gating and ordinary spreadsheet operations (MS Excel).

### Results - Image data and histograms

All stainings yielded cells that were successfully detected and quantified using the experimental system of the invention. Mouse isotype controls IgG1 and IgG2a were both negative.

In Figures 7 to 17 are shown examples of the images from the detection and quantitation of the QuantiBritePE beads and from the detection of human peripheral blood cells stained with PE conjugated antibodies.

The general impression of the images suggested that a different spacing (e.g. 30-140 µm) could be used without affecting the quantitative measurement of the cell signals.

**Table 2. Data from Figure 7 to 10. QuantiBritePE beads lot. 20977 (b) and lot. 32417 (a) in pre-diluted IgG1/PE control blood sample made as described in the "no wash" procedure. Data has been calculated and estimated using MS Excel and National Instuments LabVIEW software. Approx. 3,000 beads were detected for each data point.**

| **PE molecules** | **Log(PE molecules)** | **IFI, PBS geo Mean** | **Log (IFI, PBS geo mean)** | **IFI, IgG/PE geo Mean** | **Log(IFI, IgG/PE, geo mean)** | |
|---|---|---|---|---|---|---|
| 13,400 | 4.1271 | 18 | 1.2553 | 20 | 1.3010 | a |
| 14,000 | 4.1461 | 19 | 1.2788 | 17 | 1.2304 | b |
| 36,600 | 4.5635 | 51 | 1.7076 | 51 | 1.7076 | b |
| 37,000 | 4.5682 | 53 | 1.7243 | 62 | 1.7924 | a |
| 78,000 | 4.8921 | 120 | 2.0792 | 150 | 2.1761 | a |
| 182,000 | 5.2601 | 290 | 2.4624 | 305 | 2.4843 | b |

The IgG2a/PE stained mouse isotype control sample gave similar results as for the mouse isotype control IgG1/PE (Figure 17). Figure 17 shows the background level that defines the detection limit of the system.

Based on the data in figures 13 to 16 the following summary of the differential blood cell count have been made:

**Table 3. The normal range of positive cells is roughly calculated from data from Hall (1991) and Kuby (1994) and from Winthereik, M.P. (1995). Hall, R. and Malia, G. (1991) Medical Laboratory Haematology, Butterworth Heinemann, Oxford Press.; Kuby, J. (1994) Immunology, W.H.Freeman and Co.; Winthereik, M.P. (1995, DAKO A/S, Denmark). Unpublished reference data, DAKO production QC.**

| **Marker type** | **Number of cells** | **Volume blood analyzed** | **Measured cells/µL x 10³** | **Measured % of Leukocyte s** | **Normal range % of Leukocytes** |
|---|---|---|---|---|---|
| CD3 | 480 | 0.625 µL | approx. 0.77 | approx.22 % | 21 - 28% |
| CD4 | 202 | 0.313 µL | approx. 0.65 | approx.19 % | 18 - 35% |
| CD8 | 410 | 1.250 µL | approx. 0.33 | approx. 9 % | 8 - 20% |
| CD45 | 2200 | 0.625 µL | approx. 3.50 | - | - |

### Conclusion

The experimental fluorescence system according to the invention for detection of fluorescent objects can be used to detect and quantify fluorescent molecules on human peripheral blood cells using a common "no-wash" immuno-staining protocol for flow cytometry. The measured differential percentage counts (% of lymphocytes) on human leukocytes appear to be within the normal range for such analyses.

Furthermore antibody binding capacities (ABC) for different CD markers of the different cell types may be estimated by using calibration curves similar to the one constructed here, and by using antibodies with known fluorochrome/antibody ratios. Also a volumetric differential counting of immunostained cells seems to be possible with further development of the technology.

The detection limit and sensitivity and general performance of the system can be further increased by optimisation of the optics, electronics, chemistry and fluid system designs. It is very likely that the system can be used in many applications for the field of human diagnostics and similar fields e.g. food analysis industry, water analysis industry and related fields (see list below).

Diagnostic technologies for implication of the technology according to the invention:
- Detection/quantification of surface molecules at the level of individual cells or particles e.g. CD45, CD3, CD4, CD8, CD22, CD34, CD61
- Detection of molecules having an intracellular origin (e.g. proteins or nucleic acids)
- Detection of molecules being markers for abnormal cells or abnormal conditions
- Detection of variations in the concentration of nucleated cells in blood or body fluids (e.g. increased concentration of nucleated RBC in blood)
- Detection of bacteria or components with relation to bacteria

Some of the clinical applications that may utilise the technology:
- Differential counting of immnuno-stained leukocytes
- Immunophenotypic analysis of leukemias and lymphomas
- Detection of minimal residual disease
- Stem cell enumeration
- Viral infections
- Immunodeficiency diseases
- Reticulocyte analysis
- Platelet function
- Measurement of phagocytosis
- Measuring expression of cell cycle related proteins

### Example 2

### Investigation of the feasibility of the present invention for the assessment of particles with limited number of analyte positions

A series of testing of the feasibility of detecting particles with analytes, in a amount of less than 1x10⁶ analyte detectable positions were conducted. In all tests the detection system was equivalent to the system presented in Fig. 24, configured for transversal magnification of approximately 1.

### Test 1 - PE beads

Detection of PE was investigated using QuantiBrite PE beads (BD, USA Cat.No. 340 495) suspended in: PBS with 0.2% L31, block polymer surfactant (BASF chemicals, Germany). For excitation were used LED's emitting at 517 mn (Nichia, Japan, P/N NSPG300A), filtered with SWP 530 mn (Ferroperm, Denmark). The emitted fluorescence was filtered with 565ALP (Omega Optical, USA).

The estimated detection limit for PE was 2780 MESF PE in PBS.

### Test 2 - FITC beads

Detection of FITC was investigated using Quantum 25 FITC beads, High level (BANGs labs. cat. no.825, 7.30*µ*m) suspened in: PBS with 0.2% L31, block polymer surfactant (BASF chemicals, Germany). For excitation were used LED's emitting at 470 mn (Nichia, Japan, P/N NSPB300A), filtered with SWP475 (Ferroperm, Denmark). The emitted fluorescence was filtered with 520DF40, XF3003 (Omega Optical, USA)

The estimated detection limit for FITC was 10,000 MESF FITC in PBS.

### Test 3 - PE-Cy5 beads

Detection of PE-Cy5 was investigated using Quantum 25 PE-Cy5, Medium level (Bangs labs. cat. no. 828, 7.40*µ*m) suspened in: PBS with 0.2% L31, block polymer surfactant (BASF chemicals, Germany). For excitation were used LED's emitting at 517 mn (Nichia, Japan, P/N NSPG300A), filtered with SWP 530 mn (Ferroperm, Denmark). The emitted fluorescence was filtered with LWP 650 (Ferroperm, Denmark)

The estimated detection limit for PE-Cy5 was 750 MESF PE-Cy5 in PBS.

## Claims

1. A method for assessing at least one quality parameter or at least one quantity parameter of a particle in a liquid material,
comprising:
mixing the liquid material, said liquid material comprising particles having bound thereto or comprised therein at least one species of analytes in an amount of less than 1x10⁶ analyte detectable positions per particle, with at least one reagent material, said reagent material at least comprising a first targeting species capable of selectively and directly binding to an analyte position of said species of analytes and a labelling agent, wherein said labelling agent is a compound capable of emitting, absorbing, attenuating or scattering electromagnetic radiation to result in the generation of a detectable electromagnetic signal, wherein the first targeting species and the labelling agent are covalently or non-covalently coupled to each other,
arranging a volume of 0.1 to 100 µl of a liquid material comprising at least part of the mixture of the liquid material and the reagent material in a sample compartment having an average thickness between 20 and 200 µm and a wall part defining an exposing area, the wall part allowing electromagnetic signals from the sample in the compartment to pass through the wall to the exterior,
exposing, onto an array of active detection elements, electromagnetic signals having passed through the wall part from the liquid sample at stand still in the sample compartment, thereby obtaining an image, wherein the ratio of the linear dimension of the image on the array of detection elements to the original linear dimension in the exposing domain is smaller than 20:1,
detecting the image as intensities by individual active detection elements,
processing the intensities in order to identify representations of electromagnetic signals from the particles as distinct from representations of electromagnetic signals from background, and
obtaining the at least one quality parameter or at least one quantity parameter from the result of the processing.

2. The method according to claim 1, wherein the particle is selected from cells, cell walls, bacteria, plasmodia, virus, prions, or fragments or clusters thereof, and macromolecules and beads.

3. The method according to claim 2, whereby the particle is a bead, to which analytes are bound.

4. The method according to any of the preceding claims, whereby the analyte is selected from proteins, polypeptides, peptides, lipids, carbohydrates, lipoproteins, carbohydrate-conjugated proteins, membrane constituents, receptors, genes, DNA, RNA, or fragments or clusters thereof.

5. The method according to any of the claims 2-4, whereby the analyte is bound to a cell membrane or cell nucleus membrane, such as whereby the analyte is a cell receptor.

6. The method according to any of the claims 2-4, whereby the analyte is comprised in a cell.

7. The method according to claim 6, whereby the analyte is comprised inside an organelle.

8. The method according to claim 6, whereby the analyte is located on the surface of an organelle.

9. The method according to any of the preceding claims, whereby the particles have bound thereto or comprised therein at least one species of analytes in an amount of less than 5x10⁵ analyte detectable positions per particle, such as less than 1x10⁵ analyte detectable positions per particle, such as less than 5x10⁴ analyte detectable positions per particle, such as less than 1x10⁴ analyte detectable positions per particle, such as less than 5x10³ analyte detectable positions per particle, such as less than 1x10³ analyte detectable positions per particle, such as less than 5x10² analyte detectable positions per particle, such as less than 1x10² analyte detectable positions per particle, such as less than 10 analyte detectable positions per particle, such as 1 or 2 analyte detectable position per particle.

10. The method according to any of the preceding claims, whereby the particles in average for the population have between 500 and 50,000 analyte detectable positions per particle, more preferably in average for the population between 1,000 and 10,000 analyte detectable positions per particle bound thereto or comprised therein.

11. The method according to claim 2 or any of claims 4 to 10, wherein the cells are selected from mammalian cells, insect cells, reptile cells, fish cells, yeast cells, and fungi cells.

12. The method according to claim 2 or any of claims 4 to 11, wherein the cells are selected from blood cells, sperm cells, and bone marrow cells.

13. The method according to any of the preceding claims, whereby the liquid material comprises at least two different species of particles.

14. The method according to claim 13, whereby only one of the species of particles has bound thereto or comprised therein the species of analyte.

15. The method according to any of the preceding claims, comprising binding at least two distinct targeting species to at least two distinct species of analyte and labelling the at least two distinct targeting species with two distinct labelling agents.

16. The method according to any of the preceding claims, whereby one species of analyte is selected from CD (Cluster of Differentiation) markers, such as CD3, CD4, CD8, CD16, CD19, CD22, CD34, CD45, CD61, and CD91, Epithelial Membrane Antigen (EMA), Estrogen receptor α (ERα), Cytokeratin Human, Cytokeratin 7, Cytokeratin 20, Ki-67/PI, Phosphatidylserine, BCL2 Oncoprotein, suPAR (soluble urokinase Plasminogen Activator Receptor), urokinase, a hormone bound to a receptor, a cell cycle related protein, a marker of apoptosis, and Green fluorescent protein (GFP).

17. The method according to any of the preceding claims, whereby one species of analyte is selected from a chromosomal DNA sequence, a mitochondrial DNA sequence, a chloroplast DNA sequence, a mRNA sequence, a rRNA sequence, a nucleotide sequence comprising a single nucleotide polymorphism.

18. The method according to any of the preceding claims, whereby one species of analyte is a cell cycle related protein, e.g. cycline, such as cyclin D1, tumor suppresser protein, e.g. p53 protein, Epidermal Growth Factor protein (EGF protein), Transforming Growth Factor beta (TGF-beta1), Ki-67 protein.

19. The method according to any of the preceding claims, whereby the analyte ia a cell cycle related protein receptor such as Epidermal Growth Factor Receptor (EGFR), Cyclin Dependent Kinases, such as CDK4.

20. The method according to any of the preceding claims, whereby one species of analyte is a marker of apoptosis, e.g. membrane bound phosphatidylserines, phosphatidylserines targeted with Annexin V, BCL2 oncoprotein.

21. The method according to any of the preceding claims, whereby the at least one species of analyte is a medical marker of a disease.

22. The method according to any of the preceding claims, whereby the reagent material comprises more than one first targeting species, each of said targeting species being directed to a different analyte.

23. The method according to any of the preceding claims, whereby the targeting species is an antibody directed to the analyte species, such as an antibody selected from a monoclonal antibody, a polyclonal antibody and/or a chimeric or synthetic antibody.

24. The method according to any of the preceding claims, whereby the targeting species is a nucleotide probe complementary to a sequence of an analyte species.

25. The method according to any of the preceding claims, whereby the targeting species is an In situ hybridisation (ISH) probe, preferably a fluorescent in situ hybridisation probe (FISH).

26. The method according to any of the preceding claims, wherein the liquid material is selected from body fluids, such as blood, urine, saliva, bile, sperm, faeces, cerebro-spinal fluid, nasal secrete, tears, bone marrow, and milk, milk products, waste water, process water drinking water, food, feed, and mixtures, dilutions, or extracts thereof.

27. The method according to any of the preceding claims, wherein the reagent material further comprises lysing agents and tissue fixative agents.

28. The method according to any of the preceding claims, wherein the reagent material further comprises fluorescence quenching agents, light absorbing agents, fluorescence amplification agents, such as fluorescyl-tyramine or Cy3-tyramine.

29. The method according to any of the preceding claims, whereby the labelling agent is selected from agents giving rise to one or several of the following phenomena: attenuation of electromagnetic radiation, photoluminescence when illuminated with electromagnetic radiation, scatter of electromagnetic radiation, raman scatter.

30. The method according to claim 29, whereby the labelling agent is selected from fluorescein (FITC), phycoerythrin (RPE or PE), cyanine dyes (Cy dyes), Cy3, Cy5, Cy5.5, allophycocyanines (APC), indotrimethinecyanines, indopentamethinecyanines, acridine orange, thiazole orange, DAPI, propidium iodide (PI), ethidium iodide, 7-aminoactinomycin D, Per CP or chemically coupled combinations thereof.

31. The method according to any of the preceding claims, whereby the recording of image comprises the use of a confocal scanner.

32. The method according to any of the preceding claims, whereby the image is recorded using an array of detection devices.

33. The method according to any of the preceding claims, wherein the image is recorded using a CCD, a CMOS, a video camera or a photon counting camera.

34. The method according to any of the preceding claims, whereby the image is recorded without enlargement.

35. The method according to any of the preceding claims, whereby the enlargement ratio is below 10, more preferably below 5, such as 4, more preferably below 4 such as 2, more preferably below 2, such as 1.

36. The method according to any of the preceding claims whereby the image is recorded in one exposure.

37. The method according to any of the claims 1-35 whereby the image is recorded in two, three or more exposures.

38. The method according to claim 37, wherein the assessment of the number of particles is obtained on the basis of more than one image, preferably two images, more preferably more than two images, more preferably more than four images.

39. The method according to claim 37, where information about the changes in the image in course of time is used in the assessment of the number of particles.

40. The method according to any of the preceding claims, whereby a distinction between at least two spectral properties of a labelling agent is used to obtain the at least one quality parameter or at least one quantity parameter of the particles.

41. The method according to any of the preceding claims, whereby the recording of an image further comprises exposing a first surface of the sample directly with excitation light from a first light means having at least a first light source, by use of focusing means detecting a fluorescence signal from the first surface of the sample onto a first detection means comprising at least a first detector.

## Patentansprüche

1. Verfahren zur Bewertung zumindest eines Qualitätsparameters oder zumindest eines Quantitätsparameters eines Partikels in einem flüssigen Material,
aufweisend:
Mischen des flüssigen Materials, wobei das flüssige Material Partikel aufweist, an denen wenigstens eine Analyt-Spezies gebunden ist oder die diese darin enthalten, in einer Menge von weniger als 1x10⁶ Analyt detektierbaren Positionen pro Partikel, mit wenigstens einem Reagensmaterial, wobei das Reagensmaterial wenigstens eine erste Zielspezies, die sich wahlweise und direkt an einer Analytposition der Analyt-Spezies binden kann, und ein Markierungsmittel aufweist, wobei das Markierungsmittel ein Stoff ist, der elektromagnetische Strahlung emittieren, absorbieren, dämpfen und zerstreuen kann, so dass ein detektierbares elektromagnetisches Signal erzeugt wird, wobei die erste Zielspezies und das Markierungsmittel kovalent oder nicht-kovalent miteinander verbunden sind,
Anordnen eines Volumens von 0,1 bis 100 µl eines flüssigen Materials aufweisend wenigstens einen Teil der Mischung des flüssigen Materials mit dem Reagensmaterial in einem Probenraum mit einer durchschnittlichen Dicke von zwischen 20 und 200 µm und einem Wandabschnitt, der einen exponierbaren Bereich aufweist, wobei der Wandabschnitt es ermöglicht, dass elektromagnetische Signale von der Probe in dem Raum durch die Wand nach außen dringen können,
Projizieren, auf eine Anordnung von aktiven Detektorelementen, von elektromagnetischen Signalen, die von der flüssigen Probe bei Stillstand durch den Wandabschnitt in dem Probenraum gedrungen sind, um eine Abbildung zu erhalten, wobei das Verhältnis der linearen Abmessung der Abbildung auf der Anordnung von Detektorelementen zur originalen linearen Abmessung in dem Projektiosfeld kleiner als 20:1 ist,
Detektieren der Abbildung als Intensitäten durch die einzelnen aktiven Detektorelemente,
Verarbeiten der Intensitäten, um die Darstellungen der elektromagnetischen Signale der Partikel als Unterschied zu den Darstellungen der elektromagnetischen Hintergrundsignale zu identifizieren, und
Erhalten des wenigstens einen Qualitätsparameters oder des wenigstens einen Quantitätsparameters aus dem Ergebnis der Verarbeitung.

2. Verfahren nach Anspruch 1, bei dem das Partikel ausgewählt wird aus einer Gruppe bestehend aus Zellen, Zellwänden, Bakterien, Plasmodien, Viren, Prionen, oder Fragmenten oder Cluster davon, und Makromolekülen und Perlen.

3. Verfahren nach Anspruch 2, bei dem das Partikel eine Perle ist, an der die Analyten gebunden sind.

4. Verfahren nach einem der vorhergehenden Ansprüche, bei dem das Analyt ausgewählt wird aus einer Gruppe bestehend aus Proteinen, Polypeptiden, Peptiden, Lipiden, Kohlenhydraten, Lipoproteinen, kohlenhydrat-konjugierten Proteinen, Membranbestandteilen, Rezeptoren, Genen, DNA, RNA, oder Fragmenten oder Cluster davon.

5. Verfahren nach einem der Ansprüche 2 bis 4, bei dem das Analyt an einem Zellmembran oder einem Zellkernmembran gebunden ist, wobei das Analyt ein Zellenrezeptor ist.

6. Verfahren nach einem der Ansprüche 2 bis 4, bei dem das Analyt in einer Zelle enthalten ist.

7. Verfahren nach Anspruch 6, bei dem das Analyt in einer Organelle enthalten ist.

8. Verfahren nach Anspruch 6, bei dem sich das Analyt an der Oberfläche einer Organelle befindet.

9. Verfahren nach einem der vorhergehenden Ansprüche, bei dem an den Partikeln wenigstens eine Analyt-Spezies gebunden oder die Partikel diese darin enthalten, und zwar in einer Menge von weniger als 5x10⁵ Analyt detektierbaren Positionen pro Partikel, wie beispielsweise weniger als 1x10⁵ Analyt detektierbaren Positionen pro Partikel, wie beispielsweise weniger als 5x10⁴ Analyt detektierbaren Positionen pro Partikel, wie beispielsweise weniger als 1x10⁴ Analyt detektierbaren Positionen pro Partikel, wie beispielsweise weniger als 5x10³ Analyt detektierbaren Positionen pro Partikel, wie beispielsweise weniger als 1x10³ Analyt detektierbaren Positionen pro Partikel, wie beispielsweise weniger als 5x10² Analyt detektierbaren Positionen pro Partikel, wie beispielsweise weniger als 1x10² Analyt detektierbaren Positionen pro Partikel, wie beispielsweise weniger als 10 Analyt detektierbaren Positionen pro Partikel, wie beispielsweise 1 oder 2 Analyt detektierbaren Positionen pro Partikel.

10. Verfahren nach einem der vorhergehenden Ansprüche, bei dem die Partikel im Mittel für die Population zwischen 500 und 50.000 Analyt detektierbaren Positionen pro Partikel, vorzugsweise im Mittel für die Population zwischen 1.000 und 10.000 Analyt detektierbaren Positionen pro Partikel haben, die daran gebunden oder darin enthalten sind.

11. Verfahren nach Anspruch 2 oder einem der Ansprüche 4 bis 10, bei dem die Zellen ausgewählt werden aus einer Gruppe bestehend aus Säugetierzellen, Insektenzellen, Reptilienzellen, Fischzellen, Hefezellen und Pilzzellen.

12. Verfahren nach Anspruch 2 oder einem der Ansprüche 4 bis 11, bei dem Zellen ausgewählt sind aus einer Gruppe bestehend aus Blutzellen, Spermienzellen, und Knochenmarkzellen.

13. Verfahren nach einem der vorhergehenden Ansprüche, bei dem das flüssige Material wenigstens zwei verschiedene Partikelspezies enthält.

14. Verfahren nach Anspruch 13, bei dem nur an einer Partikelspezies die Analyt-Spezies gebunden ist oder nur eine Partikelspezies diese enthält.

15. Verfahren nach einem der vorhergehenden Ansprüche, aufweisend das Binden von wenigstens zwei verschiedenen Zielspezies an wenigstens zwei verschiedenen Analyt-Spezies, und das Markieren der wenigstens zwei verschiedenen Zielspezies mit zwei verschiedenen Markierungsmitteln.

16. Verfahren nach einem der vorhergehenden Ansprüche, bei dem eine Analyt-Spezies ausgewählt wird aus einer Gruppe bestehend aus CD (Cluster of Differentiation)-Markern, wie etwa CD3, CD4, CD8, CD16, CD19, CD22, CD34, CD45, CD61, und CD91, Ephitel-Membran-Antigen (EMA), Östrogenrezeptor α (ERα), menschliches Cytokeratin, Cytokeratin 7, Cytokeratin 20, Ki-67/PI, Phosphatidylserin, BCL2 Oncoprotein, suPAR (löslicher Urokinase-Typ Plasminogen Aktivator Rezeptor), Urokinase, rezeptorgebundenes Hormon, ein Zellzyklus bezogenes Protein, einen Marker von Apoptose, und ein grün fluoreszierendes Protein (GFP).

17. Verfahren nach einem der vorhergehenden Ansprüche, bei dem eine Analyt-Spezies ausgewählt wird aus einer Gruppe bestehend aus einer Chromosomen-DNA-Sequenz, einer mitochondrialen DNA-Sequenz, einer Chloroplast-DNA-Sequenz, einer mRNA-Sequenz, einer rRNA-Sequenz, einer Nukleotid-Sequenz, die ein Einzelnukleotid-Polymorphismus aufweist.

18. Verfahren nach einem der vorhergehenden Ansprüche, bei dem eine Analyt-Spezies ein Zellkern bezogenes Protein, wie z.B. Cyclin wie etwa Cyclin D1, ein Tumorunterdrückungs-Protein, wie z.B. das p53-Protein, ein Epidermiswachstumsfaktor-Protein (EGF-Protein), ein Protein der Transformierenden Wachstumsfaktor Beta - Familie (TGF-beta1), ein Ki-67-Protein, ist.

19. Verfahren nach einem der vorhergehenden Ansprüche, bei dem das Analyt ein Zellzyklus bezogener Proteinrezeptor, wie z.B. der Epidermiswachstumsfaktor-Rezeptor (EGFR), Cyclin-abhängige Kinasen, wie etwa CDK4, ist.

20. Verfahren nach einem der vorhergehenden Ansprüche, bei dem eine Analyt-Spezies ein Marker von Apoptose, wie z.B. ein membrangebundenes Phosphatidylserin, auf Annexin V ausgerichtetes Phosphatidylserin , BCL2 Oncoprotein, ist.

21. Verfahren nach einem der vorhergehenden Ansprüche, bei dem wenigstens eine Analyt-Spezies ein medizinischer Marker für eine Krankheit ist.

22. Verfahren nach einem der vorhergehenden Ansprüche, bei dem das Reagensmaterial mehr als eine erste Zielspezies aufweist, wobei jede Zielspezies auf ein anderes Analyt gerichtet ist.

23. Verfahren nach einem der vorhergehenden Ansprüche, bei dem die Zielspezies ein Antikörper ist, der auf die Analyt-Spezies gerichtet ist, wie etwa ein Antikörper, ausgewählt aus einer Gruppe bestehend aus einem monoclonalen Antikörper, einem polyclonalen Antikörper und/oder einem chimerischen oder synthetischen Antikörper.

24. Verfahren nach einem der vorhergehenden Ansprüche, bei dem die Zielspezies eine zu einer Sequenz einer Analyt-Spezies komplementäre Nukleotid-Probe ist.

25. Verfahren nach einem der vorhergehenden Ansprüche, bei dem die Zielspezies eine In-situ-Hybridisierung (ISH), vorzugsweise eine Fluoreszenz-in-situ-Hybridisierung (FISH) ist.

26. Verfahren nach einem der vorhergehenden Ansprüche, bei dem das flüssige Material ausgewählt ist aus einer Gruppe bestehend aus Körperflüssigkeiten, wie etwa Blut, Urin, Speichel, Galle, Sperma, Kot, Cerebrospinalflüssigkeit, Nasensekret, Tränenflüssigkeit, Knochenmark, sowie Milch, Milchprodukte, Abwasser, aufbereitetes Trinkwasser, Nahrungsmittel, Futter, und Mischungen, Lösungen oder Extrakte davon.

27. Verfahren nach einem der vorhergehenden Ansprüche, bei dem das Reagensmaterial weiterhin Lysemittel und gewebefixierende Mittel aufweist.

28. Verfahren nach einem der vorhergehenden Ansprüche, bei dem das Reagensmaterial weiterhin Fluorescence-Quenching-Mittel, lichtabsorbierende Mittel, fluoreszenzverstärkende Mittel, wie z.B. Fluorescyl-Tyramin oder Cy3-Tyramin, aufweist.

29. Verfahren nach einem der vorhergehenden Ansprüche, bei dem das Markierungsmittel ausgewählt wird aus Mitteln, die eines oder mehrere der nachstehenden Phänomene verursachen: Dämpfung der elektromagnetischen Strahlung, Photolumineszenz bei Illuminierung mit elektromagnetischer Strahlung, Zerstreuung von elektromagnetischer Strahlung, Raman-Streuung.

30. Verfahren nach Anspruch 29, bei dem das Markierungsmittel ausgewählt wird aus einer Gruppe bestehend aus Fluoreszein (FITC), Phycoerythrin (RPE oder PE), Cyaninfarbstoffe (Cy-Farbstoffe), Cy3, Cy5, Cy5,5, Allophycocyanin (APC), Indo-Trimethincyanin, Indopentamethincyanin, Acridinorage, Thiazol Orange, DAPI, Propidiumiodid (PI), Ethidiumiodid, 7-Aminoactinomycin D, Per CP oder chemisch verbundene Kombinationen davon.

31. Verfahren nach einem der vorhergehenden Ansprüche, bei dem die Aufzeichnung der Abbildung die Verwendung eines konfokalen Scanners umfasst.

32. Verfahren nach einem der vorhergehenden Ansprüche, bei dem die Abbildung unter Verwendung eines Arrays von Detektorvorrichtungen aufgezeichnet wird.

33. Verfahren nach einem der vorhergehenden Ansprüche, bei dem die Abbildung unter Verwendung eines CCD-Sensors, eines CMOS-Sensors, einer Videokamera oder einer Photonen zählenden Kamera aufgezeichnet wird.

34. Verfahren nach einem der vorhergehenden Ansprüche, bei dem die Abbildung ohne Vergrößerung aufgezeichnet wird.

35. Verfahren nach einem der vorhergehenden Ansprüche, bei dem das Vergrößerungsverhältnis unter 10, vorzugsweise unter 5, wie beispielsweise 4, vorzugsweise unter 4, wie beispielsweise vorzugsweise unter 2, wie beispielsweise 1, ist.

36. Verfahren nach einem der vorhergehenden Ansprüche, bei dem die Abbildung in einem Belichtungsvorgang aufgezeichnet wird.

37. Verfahren nach einem der Ansprüche 1 bis 35, bei dem die Abbildung in zwei, drei oder mehreren Belichtungsvorgängen aufgezeichnet wird.

38. Verfahren nach Anspruch 37, bei dem die Bestimmung der Anzahl von Partikeln auf der Grundlage von mehr als einer Abbildung, vorzugsweise von zwei Abbildungen, vorzugsweise von mehr als zwei Abbildungen, und vorzugsweise von mehr als vier Abbildungen, erfolgt.

39. Verfahren nach Anspruch 37, bei dem Information über die Veränderungen in der Abbildung über die Zeit bei der Bestimmung der Anzahl von Partikeln verwendet wird.

40. Verfahren nach einem der vorhergehenden Ansprüche, bei dem eine Unterscheidung zwischen wenigstens zwei spektralen Eigenschaften eines Markierungsmittels genutzt wird, um wenigstens einen Qualitätsparameter oder wenigstens einen Quantitätsparameter der Partikel zu erhalten.

41. Verfahren nach einem der vorhergehenden Ansprüche, bei dem die Aufzeichnung einer Abbildung weiterhin das Belichten einer ersten Oberfläche der Probe direkt mit Anregungslicht von einem ersten Leuchtmittel, das wenigstens eine erste Lichtquelle hat, umfasst, durch die Verwendung einer Fokussiereinrichtung, die ein fluoreszierendes Signal von der ersten Oberfläche der Probe auf eine erste Detektoreinrichtung, die wenigstens einen Detektor aufweist, fokussiert.

## Revendications

1. Procédé d'évaluation d'au moins un paramètre de qualité ou d'au moins un paramètre de quantité d'une particule dans un matériau liquide,
comprenant :
le mélange du matériau liquide, ledit matériau liquide comprenant des particules auxquelles est reliée ou qui comprennent au moins une espèce d'analytes en une quantité inférieure à 1 x 10⁶ positions détectables d'analytes par particule, avec au moins un matériau réactif, ledit matériau réactif comprenant au moins une première espèce de ciblage capable de se lier sélectivement et directement à une position d'analyte de ladite espèce d'analytes et un agent de marquage, ledit agent de marquage étant un composé capable d'émettre, d'absorber,
d'atténuer ou de diffuser un rayonnement électromagnétique pour résulter en la génération d'un signal électromagnétique détectable, la première espèce de ciblage et l'agent de marquage étant couplés de manière covalente ou non covalente l'un à l'autre,
la disposition d'un volume de 0,1 à 100 µl d'un matériau liquide comprenant au moins une partie du mélange du matériau liquide et du matériau réactif dans un compartiment pour échantillon d'une épaisseur moyenne comprise entre 20 et 200 µm et comprenant une partie de paroi définissant une zone d'exposition, la partie de paroi permettant le passage de signaux électromagnétiques provenant de l'échantillon dans le compartiment au travers de la paroi vers l'extérieur,
l'exposition, sur un réseau d'éléments de détection actifs, de signaux électromagnétiques ayant traversé la partie de paroi à partir de l'échantillon liquide au repos dans le compartiment pour échantillon, afin d'obtenir une image, le rapport entre la dimension linéaire de l'image sur le réseau d'éléments de détection et la dimension linéaire initiale dans le domaine d'exposition étant inférieur à 20:1,
la détection de l'image sous la forme d'intensités par des éléments de détection actifs individuels,
le traitement des intensités afin d'identifier des représentations de signaux électromagnétiques issus des particules comme étant distinctes de représentations de signaux électromagnétiques de l'arrière-plan, et
l'obtention de l'au moins un paramètre de qualité ou de l'au moins unparamètre de quantité à partir du résultat du traitement.

2. Procédé selon la revendication 1, dans lequel la particule est choisie parmi les cellules, les parois cellulaires, les bactéries, les plasmodia, les virus, les prions, ou leurs fragments ou agrégats, et les macromolécules et les billes.

3. Procédé selon la revendication 2, dans lequel la particule est une bille à laquelle des analytes sont reliés.

4. Procédé selon l'une quelconque des revendications précédentes, dans lequel l'analyte est choisi parmi les protéines, les polypeptides, les peptides, les lipides, les glucides, les lipoprotéines, les protéines conjuguées à des glucides, les constituants de membranes, les récepteurs, les gènes, l'ADN, l'ARN, ou leurs fragments ou agrégats.

5. Procédé selon l'une quelconque des revendications 2 à 4, dans lequel l'analyte est relié à une membrane cellulaire ou une membrane de noyau cellulaire, l'analyte étant par exemple un récepteur cellulaire.

6. Procédé selon l'une quelconque des revendications 2 à 4, dans lequel l'analyte est compris dans une cellule.

7. Procédé selon la revendication 6, dans lequel l'analyte est compris dans un organite.

8. Procédé selon la revendication 6, dans lequel l'analyte est situé à la surface d'un organite.

9. Procédé selon l'une quelconque des revendications précédentes, dans lequel au moins une espèce d'analytes est reliée aux particules ou comprise dans celles-ci en une quantité inférieure à 5 x 10⁵ positions détectables d'analytes par particule, par exemple inférieure à 1 x 10⁵ positions détectables d'analytes par particule, par exemple inférieure à 5 x 10⁴ positions détectables d'analytes par particule, par exemple inférieure à 1 x 10⁴ positions détectables d'analytes par particule, par exemple inférieure à 5 x 10³ positions détectables d'analytes par particule, par exemple inférieure à 1 x 10³ positions détectables d'analytes par particule, par exemple inférieure à 5 x 10² positions détectables d'analytes par particule, par exemple inférieure à 1 x 10² positions détectables d'analytes par particule, par exemple inférieure à 10 positions détectables d'analytes par particule, par exemple de 1 ou 2 positions détectables d'analytes par particule.

10. Procédé selon l'une quelconque des revendications précédentes, dans lequel les particules comprennent en moyenne pour la population entre 500 et 50 000 positions détectables d'analytes par particule, de manière davantage préférée en moyenne pour la population entre 1 000 et 10 000 positions détectables d'analytes par particule, reliées à celles-ci ou comprises dans celles-ci.

11. Procédé selon la revendication 2 ou l'une quelconque des revendications 4 à 10, dans lequel les cellules sont choisies parmi les cellules mammaliennes, les cellules d'insectes, les cellules de reptiles, les cellules de poissons, les cellules de levures et les cellules de champignons.

12. Procédé selon la revendication 2 ou l'une quelconque des revendications 4 à 11, dans lequel les cellules sont choisies parmi les cellules de sang, les cellules de sperme et les cellules de moelle osseuse.

13. Procédé selon l'une quelconque des revendications précédentes, dans lequel le matériau liquide comprend au moins deux espèces de particules différentes.

14. Procédé selon la revendication 13, dans lequel l'espèce d'analyte n'est reliée à ou comprise que dans une seule des espèces de particules.

15. Procédé selon l'une quelconque des revendications précédentes, comprenant la liaison d'au moins deux espèces de ciblage distinctes à au moins deux espèces distinctes d'analyte et le marquage des au moins deux espèces de ciblage distinctes avec deux agents de marquage distincts.

16. Procédé selon l'une quelconque des revendications précédentes, dans lequel une espèce d'analyte est choisie parmi les marqueurs CD (classe de différenciation), tels que CD3, CD4, CD8, CD16, CD19, CD22, CD34, CD45, CD61 et CD91, l'antigène de membrane épithélial (EMA), le récepteur d'estrogène α (ERα), la cytokératine humaine, la cytokératine 7, la cytokératine 20, Ki-67/PI, la phosphatidylsérine, l'oncoprotéine BCL2, suPAR (récepteur d'activateur de plasminogène de type urokinase soluble), l'urokinase, une hormone reliée à un récepteur, une protéine associée au cycle cellulaire, un marqueur de l'apoptose et une protéine fluorescente verte (GFP).

17. Procédé selon l'une quelconque des revendications précédentes, dans lequel une espèce d'analyte est choisie parmi une séquence d'ADN chromosomique, une séquence d'ADN mitochondriale, une séquence d'ADN de chloroplaste, une séquence d'ARNm, une séquence d'ARNr, une séquence nucléotidique comprenant un polymorphisme nucléotidique unique.

18. Procédé selon l'une quelconque des revendications précédentes, dans lequel une espèce d'analyte est une protéine associée au cycle cellulaire, par exemple la cycline, telle que la cycline D1, une protéine suppresseuse de tumeurs, par exemple la protéine p53, la protéine du facteur de croissance épidermique (protéine EGF), le facteur de croissance transformant bêta(TGF-bêta 1), la protéine Ki-67.

19. Procédé selon l'une quelconque des revendications précédentes, dans lequel l'analyte est un récepteur d'une protéine associée au cycle cellulaire, tel que le récepteur du facteur de croissance épidermique (EGFR), les kinases dépendantes de la cycline, telles que CDK4.

20. Procédé selon l'une quelconque des revendications précédentes, dans lequel une espèce d'analyte est un marqueur de l'apoptose, p. ex. des phosphatidylsérines reliées à la membrane, des phosphatidylsérines ciblées avec l'annexine V, l'oncoprotéine BCL2.

21. Procédé selon l'une quelconque des revendications précédentes, dans lequel l'au moins une espèce d'analyte est un marqueur médical d'une maladie.

22. Procédé selon l'une quelconque des revendications précédentes, dans lequel le matériau réactif comprend plus d'une première espèce de ciblage, chacune desdites espèces de ciblage étant dirigée vers un analyte différent.

23. Procédé selon l'une quelconque des revendications précédentes, dans lequel l'espèce de ciblage est un anticorps dirigé contre l'espèce d'analyte, tel qu'un anticorps choisi parmi un anticorps monoclonal, un anticorps polyclonal et/ou un anticorps chimérique ou synthétique.

24. Procédé selon l'une quelconque des revendications précédentes, dans lequel l'espèce de ciblage est une sonde nucléotidique complémentaire d'une séquence d'une espèce d'analyte.

25. Procédé selon l'une quelconque des revendications précédentes, dans lequel l'espèce de ciblage est une sonde d'hybridation in situ (ISH), de préférence une sonde d'hybridation in situ fluorescente (FISH).

26. Procédé selon l'une quelconque des revendications précédentes, dans lequel le matériau liquide est choisi parmi les fluides corporels tels que le sang, l'urine, la salive, la bile, le sperme, les fèces, le liquide céphalorachidien, les sécrétions nasales, les larmes, la moelle osseuse, et le lait, les produits laitiers, l'eau usée, l'eau de traitement, l'eau potable, les aliments, les aliments pour animaux, et leurs mélanges, dilutions ou extraits.

27. Procédé selon l'une quelconque des revendications précédentes, dans lequel le matériau réactif comprend également des agents de lyse et des agents fixateurs de tissus.

28. Procédé selon l'une quelconque des revendications précédentes, dans lequel le matériau réactif comprend également des agents d'extinction de fluorescence, des agents absorbant la lumière, des agents d'amplification de la fluorescence, tels que la fluorescyl-tyramine ou la Cy3-tyramine.

29. Procédé selon l'une quelconque des revendications précédentes, dans lequel l'agent de marquage est choisi parmi les agents donnant lieu à un ou plusieurs des phénomènes suivants : une atténuation d'un rayonnement électromagnétique, une photoluminescence lors d'une exposition à un rayonnement électromagnétique, une diffusion d'un rayonnement électromagnétique, une diffusion Raman.

30. Procédé selon la revendication 29, dans lequel l'agent de marquage est choisi parmi la fluorescéine (FITC), la phycoérythrine (RPE ou PE), les colorants cyanine (colorants Cy), Cy3, Cy5, Cy5.5, les allophycocyanines (APC), les indotriméthinecyanines, les indopentaméthinecyanines, l'orangé d'acridine, l'orangé de thiazole, DAPI, l'iodure de propidium (PI), l'iodure d'éthidium, la 7-aminoactinomycine D, Per CP ou leurs combinaisons couplées chimiquement.

31. Procédé selon l'une quelconque des revendications précédentes, dans lequel l'enregistrement de l'image comprend l'utilisation d'un scanner confocal.

32. Procédé selon l'une quelconque des revendications précédentes, dans lequel l'image est enregistrée en utilisant un réseau de dispositifs de détection.

33. Procédé selon l'une quelconque des revendications précédentes, dans lequel l'image est enregistrée en utilisant une caméra CCD, une caméra CMOS, une caméra vidéo ou une caméra de numération de photons.

34. Procédé selon l'une quelconque des revendications précédentes, dans lequel l'image est enregistrée sans agrandissement.

35. Procédé selon l'une quelconque des revendications précédentes, dans lequel le rapport d'agrandissement est inférieur à 10, de manière davantage préférée inférieur à 5, tel que de 4, de manière davantage préférée inférieur à 4, tel que de 2, de manière davantage préférée inférieur à 2, tel que de 1.

36. Procédé selon l'une quelconque des revendications précédentes, dans lequel l'image est enregistrée en une exposition.

37. Procédé selon l'une quelconque des revendications 1 à 35, dans lequel l'image est enregistrée en deux, trois expositions ou plus.

38. Procédé selon la revendication 37, dans lequel l'évaluation du nombre de particules est obtenue à partir de plus d'une image, de préférence de deux images, de manière davantage préférée de plus de deux images, de manière davantage préférée de plus de quatre images.

39. Procédé selon la revendication 37, dans lequel des informations relatives aux modifications de l'image au cours du temps sont utilisées pour l'évaluation du nombre de particules.

40. Procédé selon l'une quelconque des revendications précédentes, dans lequel une distinction entre au moins deux propriétés spectrales d'un agent de marquage est utilisée pour obtenir l'au moins un paramètre de qualité ou l'au moins un paramètre de quantité des particules.

41. Procédé selon l'une quelconque des revendications précédentes, dans lequel l'enregistrement d'une image comprend également l'exposition d'une première surface de l'échantillon directement à une lumière d'excitation issue d'un premier moyen d'éclairage comprenant au moins une première source de lumière, en utilisant un moyen de focalisation, la détection d'un signal de fluorescence issu de la première surface de l'échantillon sur un premier moyen de détection comprenant au moins un premier détecteur.
